# EUROPEAN PATENT APPLICATION

(11) **EP 4 122 951 A1**
(43) Date of publication of application: **25.01.2023**
(21) Application number: 21770699.3
(22) Date of filing: 19.03.2021
(51) Int. Cl.: C07K 14/55, C12N 15/26, C12N 15/63, A61K 38/20, A61P 35/00

(54) **INTERLEUKIN-2 MUTANT AND USE THEREOF**

(30) Priority: 19.03.2020 CN 202010196689
(71) Applicant: Innovent Biologics (Suzhou) Co., Ltd., Suzhou, Jiangsu 215123 (CN)
(72) Inventor: FU, Fenggen, Suzhou, Jiangsu 215123 (CN); ZHOU, Shuaixiang, Suzhou, Jiangsu 215123 (CN); WU, Weiwei, Suzhou, Jiangsu 215123 (CN); HE, Kaijie, Suzhou, Jiangsu 215123 (CN); LIU, Junjian, Suzhou, Jiangsu 215123 (CN)
(74) Representative: Teuten, Andrew John
(86) International application number: PCT/CN2021/081841
(87) International publication number: WO 2021/185362

(57) **Abstract**

The present invention relates to a novel interleukin-2 (IL-2) mutant protein. The present invention further provides a fusion protein and an immunoconjugate comprising the IL-2 mutant protein, a nucleic acid encoding the IL-2 mutant protein, and a vector and a host cell comprising the nucleic acid. The present invention further provides a method for preparing the IL-2 mutant protein, a pharmaceutical composition comprising the IL-2 mutant protein, and therapeutic use of the mutant protein.

## Description

### TECHNICAL FIELD

The present invention relates to a novel interleukin-2 (IL-2) mutant protein and use thereof. In particular, the present invention relates to an IL-2 mutant protein with improved properties, such as improved druggability, reduced binding ability for an IL-2Rα receptor, and/or enhanced binding ability for an IL-2RP receptor, compared to a wild-type IL-2 protein. The present invention further provides a fusion protein and an immunoconjugate comprising the IL-2 mutant protein, a nucleic acid encoding the IL-2 mutant protein, and a vector and a host cell comprising the nucleic acid. The present invention further provides a method for preparing and screening the IL-2 mutant protein, a pharmaceutical composition comprising the IL-2 mutant protein, and therapeutic use of the mutant protein.

### BACKGROUND

Interleukin-2 (IL-2), also known as T-cell growth factor (TCGF), is a multifunctional cytokine produced mainly by activated T cells, particularly by CD4⁺T helper cells. In eukaryotic cells, human IL-2 (UniProt: P60568) is synthesized as a precursor polypeptide of 153 amino acids, and mature secretory IL-2 is produced after removal of 20 N-terminus amino acids. The sequences of IL-2 from other species have also been disclosed. See NCBI Ref Seq No. NP032392 (mice), NP446288 (rats) or NP517425 (chimpanzees).

Interleukin-2 has 4 antiparallel and amphipathic α helices, which form a quaternary structure essential for its function (Smith, Science 240,1169-76 (1988); Bazan, Science 257,410-413 (1992)). In most cases, IL-2 acts through three different receptors: interleukin-2 receptor α (IL-2Rα; CD25), interleukin-2 receptor β (IL-2RP; CD122), and interleukin-2 receptor γ (IL-2Ry; CD132). IL-2RP and IL-2Rγ are critical for IL-2 signaling, while IL-2Rα (CD25) is not essential for signaling but can enable IL-2 to bind to a receptor with high affinity (Krieg et al., Proc Natl Acad Sci 107,11906-11 (2010)). The trimeric receptor (IL-2αβγ) formed by the combination of IL-2Rα, IL-2RP, and IL-2Rγ is an IL-2 high-affinity receptor (with a K_{D} of about 10 pM), the dimeric receptor (IL-2βγ) consisting of IL-2RP and IL-2Rγ is an intermediate affinity receptor (with a K_{D} of about 1 nM), and the IL-2 receptor formed solely by subunit α is a low affinity receptor.

Immune cells express dimeric or trimeric IL-2 receptors. The dimeric receptor is expressed on cytotoxic CD8⁺ T cells and natural killer (NK) cells, whereas the trimeric receptor is expressed predominantly on activated lymphocytes and CD4⁺ CD25⁺ FoxP3⁺ suppressive regulatory T cells (Treg) (Byman, O. and Sprent. J. Nat. Rev. Immunol. 12, 180-190 (2012)). Effector T cells and NK cells in a resting state are relatively insensitive to IL-2 because they do not have CD25 on the cell surface. However, Treg cells consistently express the highest level of CD25 *in vivo,* and therefore normally IL-2 would preferentially stimulate Treg cell proliferation.

IL-2 mediates multiple actions in an immune response by binding to IL-2 receptors on different cells. In one aspect, as an immune system stimulator, IL-2 can stimulate T cell proliferation and differentiation, induce cytotoxic T lymphocyte (CTL) production, promote B cell proliferation and differentiation and immunoglobulin synthesis, and stimulate the production, proliferation and activation of natural killer (NK) cells, and thus has been approved as an immunotherapeutic agent for the treatment of cancer and chronic viral infection. In another aspect, IL-2 contributes to the maintenance of immunosuppressive CD4⁺ CD25⁺ regulatory T cells (i.e., Treg cells) (Fontenot et al., Nature Immunol 6,1142-51 (2005); D'Cruz and Klein, Nature Immunol 6,1152-59 (2005); Maloy and Powrie, Nature Immunol 6,1171-72 (2005)), and mediates activation-induced cell death (AICD) and participates in the establishment and maintenance of immune tolerance to autoantigens and tumor antigens (Lenardo et al., Nature 353:858 (1991)), thus causing, in patients, tumor tolerance due to AICD and immunosuppression due to activated Treg cells. In addition, high-dose IL-2 administration may cause vascular leak syndrome (VLS) in patients. IL-2 has been shown to induce pulmonary edema by direct binding to IL-2 trimeric receptors (IL-2αβγ) on lung endothelial cells (Krieg et al., Proc Nat Acad Sci USA 107,11906-11 (2010)). To solve the above problems associated with IL-2 immunotherapy, it has been proposed to alter the selectivity or preference of IL-2 for different receptors to reduce the toxicity of IL-2 therapy and/or improve its effect. For example, it has been proposed that a complex of a monoclonal antibody and IL-2, by targeting IL-2 to cells expressing CD122 but not CD25, induces preferential amplification of CD122^{high} populations, and improves the effect of IL-2 therapy *in vivo* (Boyman et al., Science 311, 1924-1927 (2006)). Oliver AST et al. (US2018/0142037) proposed to introduce triple mutations F42A/Y45A/L72G at amino acid residue positions 42, 45, and 72 of IL-2 so as to reduce the affinity for the IL-2Rα receptor. Aron M. Levin et al. (Nature, Vol 484, p529-533, DOI: 10.1038/nature10975) proposed an IL-2 mutant IL-2^{H9} called "superkine", which comprises quintuple mutations L80F/R81D/ L85V/I86V/I92F and has enhanced binding to IL-2Rβ, thereby boosting the stimulation of CD25⁻cells, while still maintaining high binding to CD25. Rodrigo Vazquez-Lombardi et al. (Nature Communications, 8:15373, DOI: 10.1038/ncomms15373) proposed a human IL-2 mutant protein IL-2^{3X} with triple mutations, which has residue mutations R38D-K43E-E61R at amino acid residue positions 38, 43, and 61, respectively, resulting in the mutant protein not binding to IL-2Rα. However, the mutant protein has a weak activation effect on CD25⁻ cells, but remains activation preference for CD25⁺ cells. In addition, Rodrigo Vazquez-Lombardi et al. also proposed preparing an interleukin-2-Fc fusion to improve the pharmacodynamic properties of interleukins. However, the expression yield of the fusion protein is low, and it easily forms aggregates.

In view of the role of IL-2 in immune regulation and disease, there remains a need in the art to develop new IL-2 molecules with improved properties, particularly IL-2 molecules that are advantageous to production and purification and have improved pharmacodynamic properties.

### BRIEF SUMMARY

The present invention satisfies the above needs by providing a novel IL-2 mutant protein with improved druggability and/or improved IL-2 receptor selectivity/preference relative to wild-type IL-2 protein.

Thus, in one aspect, the present invention provides a novel IL-2 mutant protein. In some embodiments, the IL-2 mutant protein disclosed herein has one or more of the following properties, preferably at least properties (i) and (ii):
(i) improved druggability, particularly improved expression yield and/or purification performance when expressed in mammalian cells;
(ii) reduced or eliminated binding to IL-2Rα;
(iii) enhanced binding to IL-2Rβ.

In some embodiments, the present invention provides an IL-2 mutant protein comprising a mutation at the binding interface of IL-2 to IL-2Rα and having a shortened B'C' loop sequence.

In addition, the present invention provides a fusion protein and an immunoconjugate comprising the IL-2 mutant protein, a pharmaceutical composition, and a combination product; a nucleic acid encoding the IL-2 mutant protein, and a vector and a host cell comprising the nucleic acid; and a method for producing the IL-2 mutant protein, the fusion protein and the immunoconjugate disclosed herein.

Furthermore, the present invention further provides a method for treating diseases, and a method and use for stimulating the immune system in a subject using the IL-2 mutant protein, the fusion, and the immunoconjugate disclosed herein.

The present invention is further illustrated in the following drawings and specific embodiments. However, these drawings and specific embodiments should not be construed as limiting the scope of the present invention, and modifications easily conceived by those skilled in the art will be included in the spirit of the present invention and the protection scope of the appended claims.

### BRIEF DESCRIPTION OF THE DRAWINGS

FIG. 1 shows the crystal structure of a complex of IL-2 and IL-2Rα (PDB: 1Z92).
FIG. 2 shows (A) the crystal structure of IL-2 (PBD: 2ERJ) and (B) the B'C' loop structure superpose of human IL-2 and human IL-15.
FIG. 3 shows primers used to construct mutant library IBYDL029.
FIG. 4 shows IL-2 mutant proteins screened from the mutant library IBYDL029 and sequences thereof.
FIGs. 5A-B show signal curves of activation of p-STAT5 on (A) CD8⁺ CD25⁻ T cells and (B) CD8⁺ CD25⁺ T cells by selected and constructed IL-2^{mutant}-FC fusion proteins.
FIGs. 6A-C show (A) the *in vivo* anti-tumor effect of IL-2^{mutant}-FC fusion protein Y092 and (B and C) changes in the body weight monitored after administration to animals.
FIGs. 7A-C show (A) the *in vivo* anti-tumor effect of IL-2^{mutant}-FC fusion protein Y144 and (B and C) changes in the body weight monitored after administration to animals.
FIG. 8 shows the amino acid sequence of the wild-type IL-2 protein IL-2^{WT} (SEQ ID NO: 1) and the numbering of amino acid residues thereof, and shows the sequence alignment with the mutant protein IL-2^{3X}.
FIG. 9 shows the complete sequence of the yeast surface display plasmid pYDC011.

### DETAILED DESCRIPTION

Unless defined otherwise, all technical and scientific terms used herein have the same meaning as commonly understood by those of ordinary skill in the art. For the purposes of the present invention, the following terms are defined below.

The term "about" used in combination with a numerical value is intended to encompass the numerical values in a range from a lower limit less than the specified numerical value by 5% to an upper limit greater than the specified numerical value by 5%.

The term "and/or" should be understood to refer to any one of the options or any two or more of the options.

As used herein, the term "comprise" or "include" is intended to include the described elements, integers or steps, but not to exclude any other elements, integers or steps. As used herein, the term "comprise" or "include", unless indicated otherwise, also encompasses the situation where the entirety consists of the described elements, integers or steps. For example, when an IL-2 mutant protein "comprising" or "including" a mutation or a combination of mutations is mentioned, it is also intended to encompass IL-2 mutant proteins having the mutation or combination of mutations only.

As used herein, wild-type "interleukin-2" or "IL-2" refers to a parent IL-2 protein, preferably a naturally-occurring IL-2 protein, e.g., a native IL-2 protein derived from a human, mouse, rat, or non-human primate, serving as a template to which a mutation or a combination of mutations disclosed herein is introduced, including both unprocessed (e.g., without the removal of the signal peptide) and processed (e.g., with the removal of the signal peptide) forms. A full-length native human IL-2 sequence comprising a signal peptide is set forth in SEQ ID NO: 2 and the sequence of its mature protein is set forth in SEQ ID NO: 3. In addition, this term also includes naturally-occurring allelic and splice variants, isotypes, homologs, and species homologs of IL-2. This term also includes variants of native IL-2, which may, for example, have at least 95%-99% or more identity to the native IL-2 or have no more than 1-10 or 1-5 amino acid mutations (especially conservative amino acid substitutions) and preferably have substantially the same binding affinity for IL-2Rα and/or IL2RP as the native IL-2 protein. Therefore, in some embodiments, compared to the native IL-2 protein, the wild-type IL-2 protein may comprise amino acid mutations that do not affect its binding to the IL-2 receptor. For example, a native human IL-2 protein (UniProt: P60568) with a mutation C125S introduced at position 125 is a wild-type IL-2 protein disclosed herein. An example of a wild-type human IL-2 protein comprising the C125S mutation is set forth in SEQ ID NO: 1. In some embodiments, the wild-type IL-2 sequence may have at least more than 85% or 95%, or even at least 96%, 97%, 98%, or 99% amino acid sequence identity to the amino acid sequence set forth in SEQ ID NO: 1, 2, or 3. As used herein, the amino acid mutation may be an amino acid substitution, deletion, insertion, and addition. Any combination of substitution, deletion, insertion and addition may be made to obtain a final mutant protein construct with the desired properties, such as reduced binding affinity for IL-2Rα and/or improved druggability. Amino acid deletions and insertions include amino- and/or carboxyl-terminal deletions and insertions of a polypeptide sequence, as well as deletions and insertions within the polypeptide sequence. For example, an alanine residue can be deleted at position 1 of a full-length human IL-2, or one or more amino acids can be deleted from a B'C' loop region to shorten the length of the loop region. In some embodiments, the preferred amino acid mutations are amino acid substitutions, e.g., the combination of single amino acid substitutions or the replacement of segments of an amino acid sequence. For example, the entirety or a part of the B'C' loop region sequence of the wild-type IL-2 can be replaced with a different sequence, preferably to obtain a shortened B'C' loop region sequence.

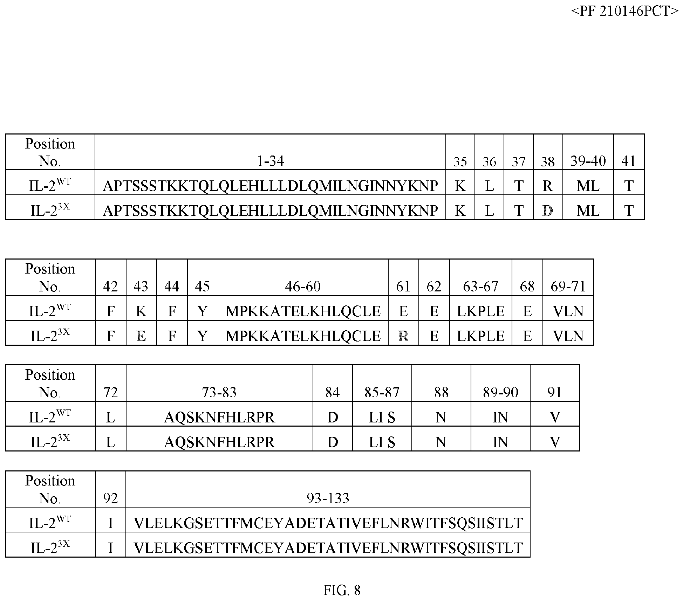

In the present invention, when the amino acid position in the IL-2 protein or IL2 sequence segments is mentioned, it is determined by referring to the amino acid sequence of the wild-type IL-2 protein (also referred to as IL-2^{WT}) set forth in SEQ ID NO: 1 (as shown in FIG. 8). The corresponding amino acid position in other IL-2 proteins or polypeptides (including full-length sequences or truncated fragments) can be identified by amino acid sequence alignment with SEQ ID NO: 1. Therefore, in the present invention, unless otherwise stated, an amino acid position in an IL-2 protein or polypeptide is an amino acid position numbered according to SEQ ID NO: 1. For example, when "F42" is mentioned, it refers to a phenylalanine residue F at position 42 of SEQ ID NO: 1, or an amino acid residue at corresponding positions in other IL-2 polypeptide sequences by alignment. To perform a sequence alignment for determining an amino acid position, Basic Local Alignment Search Tool available at https://blast.ncbi.nlm.nih.gov/Blast.cgi can be used with default parameters.

When an IL-2 mutant protein is mentioned herein, a single amino acid substitution is described as [original amino acid residue/position/amino acid residue for substitution]. For example, the substitution of lysine at position 35 with glutamate can be indicated as K35E. When there are multiple optional amino acid substitutions (e.g., D and E) at a given position (e.g., K35), the amino acid substitutions can be indicated as K35D/E. Correspondingly, single amino acid substitutions can be linked together by "+" or "-" to indicate a combinatorial mutation at multiple given positions. For example, the combinatorial mutation of positions K35E, T37E, R38E and F42A is indicated as K35E+T37E+R38E+F42A or K35E-T37E-R38E-F42A.

As used herein, the "percent sequence identity" can be determined by comparing two optimally aligned sequences over a comparison window. Preferably, the sequence identity is determined over the full length of a reference sequence (e.g., SEQ ID NO: 1). Methods of sequence alignment for comparison are well known in the art. Algorithms suitable for determining the percent sequence identity include, for example, BLAST and BLAST 2.0 algorithms (see Altschul et al., Nuc. Acids Res.25: 3389-402, 1977 and Altschul et al., J.mol. Biol. 215: 403-10, 1990). Software for performing BLAST analysis is publicly available from the National Center for Biotechnology Information. For the purpose of the present application, the percent identity can be determined by using Basic Local Alignment Search Tool available at https://blast.ncbi.nlm.nih.gov/Blast.cgi with default parameters.

As used herein, the term "conservative substitution" means an amino acid substitution that does not adversely affect or alter the biological function of a protein/polypeptide comprising an amino acid sequence. For example, a conservative substitution may be introduced by standard techniques known in the art, such as site-directed mutagenesis and PCR-mediated mutagenesis. A typical conservative amino acid substitution involves a substitution of an amino acid by another amino acid having similar chemical properties (e.g., charge or hydrophobicity). Conservative replacement tables of functionally similar amino acids are well known in the art. In the present invention, residues for conservative substitutions are from the conservative substitution table X below, particularly from the preferred residues for conservative amino acid substitutions in Table X.

**Table X**

| Original residues | Exemplary substitution | Preferred conservative amino acid substitution |
|---|---|---|
| Ala (A) | Val; Leu; Ile | Val |
| Arg (R) | Lys; Gln; Asn | Lys |
| Asn (N) | Gln; His; Asp; Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Nle | Leu |
| Leu (L) | Nle; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Nle | Leu |

For example, relative to one of SEQ ID NOs: 1-3, the wild-type IL-2 protein may have conservative amino acid substitutions, or only have conservative amino acid substitutions. As another example, the IL-2 mutant protein disclosed herein may have conservative amino acid substitutions, or only have conservative amino acid substitutions, relative to the IL-2 mutant protein sequence specifically set forth herein (e.g., any one of SEQ ID NOs: 22-26).

"Affinity" or "binding affinity" refers to the inherent binding ability that reflects the interaction between members of a binding pair. The affinity of a molecule X for its binding partner Y can be represented by an equilibrium dissociation constant (K_{D}), which is the ratio of a dissociation rate constant (k_{dis}) to an association rate constant (kₒₙ). The binding affinity can be measured by common methods known in the art. One particular method for measuring the affinity is the ForteBio affinity assay technique described herein.

As used herein, an antigen-binding molecule is a polypeptide molecule that can specifically bind to an antigen, e.g., an immunoglobulin molecule, an antibody, or an antibody fragment (e.g., an Fab fragment and an scFv fragment).

As used herein, an antibody Fc fragment refers to a C-terminus region of an immunoglobulin heavy chain that contains at least a portion of the constant region, and may include Fc fragments of native sequences and variant Fc fragments. Fc fragments of native sequences include various naturally-occurring Fc sequences of immunoglobulins, such as various Ig subclasses or the Fc regions of allotypes thereof (Gestur Vidarsson et al., IgG subclasses and allotypes: from structure to effector functions, 20 October 2014, doi: 10.3389/fimmu.2014.00520.). In one embodiment, the heavy chain Fc fragment of human IgG extends from Cys226 or Pro230 of the heavy chain to the carboxy-terminus. In another embodiment, the C-terminal lysine (Lys447) of the Fc fragment may or may not be present. In other embodiments, the Fc fragment is a variant Fc fragment comprising a mutation, for example, a L234A-L235A mutation. Unless otherwise indicated herein, amino acid residues in the Fc fragment are numbered according to the EU numbering system, also called the EU index, as described in Kabat, E.A. et al., *Sequences of Proteins of Immunological Interest,* 5th edition, Public Health Service, National Institutes of Health, Bethesda, MD (1991), NIH Publication 91-3242.

All aspects of the present invention are further detailed in the following sections.

### 1. IL-2 Mutant Protein Disclosed Herein

In one aspect, the present invention provides a novel IL-2 mutant protein with improved druggability and/or improved IL-2 receptor selectivity/preference.

### Advantageous biological properties of the IL-2 mutant protein disclosed herein

The inventors have found that the binding of the IL-2 mutant protein to IL-2Rα can be reduced or eliminated by introducing one or more specific mutations at the binding interface of the IL-2 to the IL-2Rα receptor. Furthermore, the inventors have found that the expression and/or purity of the IL-2 can be increased and/or the affinity of the IL-2 for the IL-2RP can be increased by replacing the B'C' loop sequence of the IL-2 with a short B'C' loop sequence from another interleukin cytokine such as IL-15, or by truncating the B'C' loop sequence of the IL-2. The inventors have further found that IL-2 mutations with improved properties selected from, for example, one or more of the following can be provided by combining the IL-2Rα receptor binding interface mutations with the shortened B'C' loop region mutations: (i) improved expression and/or purity; (ii) reduced or eliminated binding to IL-2Rα receptor; and/or (iii) enhanced binding to IL-2RP receptor.

Thus, the present invention provides an IL-2 mutant protein having one or more of the above-mentioned improved properties (i) to (iii), particularly having both the improved properties (i) and (ii).

### Improved druggability

In some embodiments, the IL-2 mutant protein disclosed herein has improved druggability. For example, when expressed in mammalian cells such as H293T cells or CHO cells, e.g., in the form of an Fc fusion protein, the IL-2 mutant protein has one or more properties selected from the following: (i) a superior expression yield to the wild-type IL-2 protein; and (ii) ease of purification to a higher protein purity. In some embodiments, the IL-2 mutant protein disclosed herein further has storage stability. For example, after being stored at 40 °C in PBS buffer at pH 7.4 for 14 days, the protein has a decrease of no more than 5%, 2% or 1% in purity as measured by SEC-HPLC, or has a decrease of no more than 5%, 3% or 2% in purity as measured by CE-SDS.

In some embodiments disclosed herein, the IL-2 mutant protein disclosed herein shows an increased expression level compared to the wild-type IL-2. In some embodiments disclosed herein, the increased expression occurs in a mammalian cell expression system. The expression level can be determined by any suitable method that allows for quantitative or semi-quantitative analysis of the amount of recombinant IL-2 protein in cell culture supernatant, preferably the supernatant purified by one-step affinity chromatography. For example, the amount of recombinant IL-2 protein in a sample can be assessed by Western blotting or ELISA. In some embodiments, the expression yield of the IL-2 mutant protein disclosed herein in mammalian cells is increased by at least 1.1 times, or at least 1.5 times, or at least 2 times, 3 times or 4 times or more, or at least 5, 6, 7, 8 or 9 times, or even 10 times or more, compared to that of the wild-type IL-2.

In some embodiments, as shown by determining the purity of the protein purified by protein A affinity chromatography, the IL-2 mutant protein-Fc fusion disclosed herein has higher purity, relative to the wild-type IL-2 protein fusion. In some embodiments, the purity of the protein is determined by SEC-HPLC. In some preferred embodiments, the IL-2 mutant protein disclosed herein can have a purity of up to 70%, or 80%, or 90% or higher, preferably 92%, 93%, 94%, 95%, 98% or 99% or higher, after being purified by one-step protein A affinity chromatography according to the method for purifying an IL-2 fusion protein described in Example 2.

### Improved IL-2 receptor selectivity/preference

The IL-2 protein triggers signaling and functions by interacting with IL-2 receptors. Wild-type IL-2 exhibits different affinities for different IL-2 receptors. IL-2β and IL-2γ receptors having a low affinity for wild-type IL-2 are expressed on resting effector cells, including CD8⁺ T cells and NK cells. IL-2Rα receptors having a high affinity for wild-type IL-2 are expressed on regulatory T cell (Treg) cells and activated effector cells. Due to high affinity, the wild-type IL-2 will preferentially bind to IL-2Rα on the cell surface and then recruit IL-2Rβγ. Treg cells and activated effector cells are stimulated by downstream p-STAT5 signals released through the IL-2Rβγ. Thus, without being bound by theory, reducing or eliminating the affinity of IL-2 for the IL-2Rα receptor will reduce the preference of IL-2 for preferentially activating CD25⁺ cells and the IL-2 mediated immune downregulation of Treg cells. Without being bound by theory, maintaining or enhancing the affinity for the IL-2β receptor will retain or enhance the activation of IL-2 on effector cells such as CD8⁺ T cells and NK cells, and thus the immunostimulation of IL-2.

Therefore, in some embodiments, the IL-2 mutant protein disclosed herein has improved properties relative to the wild-type IL-2, which are selected from, for example, one or more of the following:
(1) reduced or eliminated binding affinity for IL-2Rα receptor;
(2) enhanced binding affinity for IL-2RP receptor;
(3) reduced binding affinity for high-affinity IL-2R receptor (IL-2Rαβγ);
(4) increased binding affinity for intermediate-affinity IL-2R receptor (IL-2Rβγ);
(5) a reduced ability to activate IL-2 signaling, particularly STATS phosphorylation signals, in CD25⁺ cells (particularly activated CD8⁺ T cells and Treg cells);
(6) resulting in a decrease in IL-2 mediated activation and proliferation of CD25⁺ cells (particularly activated CD8⁺ T cells and Treg cells);
(7) reducing or eliminating preference of the IL-2 for preferentially stimulating Treg cell proliferation;
(8) reducing the IL-2 mediated immune downregulation effect of Treg cells;
(9) maintaining or enhancing, especially enhancing, the activation of CD25⁻ cells, particularly CD25⁻ T effector cells and NK cells; and
(10) resulting in an increase in IL-2 mediated activation and proliferation of effector T cells and NK cells.

In some embodiments, the IL-2 mutant protein disclosed herein has the property of (1) above, preferably further has one or more, especially all, properties selected from (3) and (5)-(8), and more preferably still further has one or more, especially all, properties selected from (2), (4) and (9)-(10). In some embodiments, the IL-2 mutant protein disclosed herein has the properties of (2) and (4) above, preferably further has one or more, especially all, properties selected from (9)-(10), and more preferably still further has one or more, especially all, properties selected from (1), (3), and (5)-(8).

In a preferred embodiment, the IL-2 mutant protein disclosed herein has an *in vivo* anti-tumor effect, e.g., on colon cancer.

In some preferred embodiments, the IL-2 mutant protein disclosed herein also has reduced *in vivo* toxicity mediated by the binding of IL-2 to the high-affinity receptor IL-2αβγ relative to the wild-type IL-2 protein.

In some preferred embodiments, the IL-2 mutant protein disclosed herein has no significant toxicity after being administered to a subject, as reflected, e.g., by changes in the body weight of the subject after administration. For example, after administration for a period of time, e.g., 20 days or longer, the body weight is reduced by no more than 15%, or no more than 10%, or no more than 5%, compared to the body weight before the administration.

In some embodiments, the binding affinity of the IL-2 mutant protein disclosed herein for the IL-2Rα receptor is reduced by at least 5 times, at least 10 times, or at least 25 times, particularly at least 30 times, 50 times or 100 times or more, relative to the wild-type IL-2 (e.g., IL-2^{WT} set forth in SEQ ID NO: 1). In a preferred embodiment, the mutant protein disclosed herein does not bind to the IL-2Rα receptor. The binding affinity can be determined by measuring the equilibrium dissociation constant (K_{D}) of the binding of the IL-2 mutant protein disclosed herein, such as the IL-2 mutant protein disclosed herein fused to an Fc fragment, to the IL-2Rα receptor using the ForteBio affinity assay technique.

In some embodiments, the binding affinity of the IL-2 mutant protein disclosed herein for the IL-2Rβ receptor is increased by 5-10 times or more, relative to the wild-type IL-2 (e.g., IL-2^{WT} set forth in SEQ ID NO: 1). The binding affinity can be determined by measuring the equilibrium dissociation constant (K_{D}) of the binding of the IL-2 mutant protein disclosed herein, such as the IL-2 mutant protein disclosed herein fused to an Fc fragment, to the IL-2RP receptor using the ForteBio affinity assay technique. In one embodiment, in a ForteBio affinity assay (e.g., the ForteBio affinity assay described in the examples), the bivalent binding affinity K_{D} value of the IL-2 mutant protein disclosed herein (in the form of an IL-2-Fc fusion protein) for the IL-2Rβ receptor is less than 10.0E-09 M, e.g., 1.0E-09 M to 7E-09 M, or, e.g., less than 1.0E-09 M, e.g., 1.0E-10 M to 7.0E-10 M.

In one embodiment, the IL-2 mutant protein disclosed herein reduces IL-2-mediated activation and/or proliferation of CD25⁺ cells relative to the wild-type IL-2. In one embodiment, the CD25⁺ cells are CD25⁺ CD8⁺ T cells. In another embodiment, the CD25⁺ cells are Treg cells. In one embodiment, in the STATS phosphorylation assay, the ability of the IL-2 mutant protein to activate CD25⁺ cells is identified by measuring the activation of STATS phosphorylation signals by the IL-2 mutant protein in CD25⁺ cells. For example, as described in the examples of this application, STATS phosphorylation in cells can be analyzed by flow cytometry to determine the half maximum effective concentration (EC₅₀).

In one embodiment, the IL-2 mutant protein disclosed herein maintains or enhances IL-2-mediated activation and/or proliferation of CD25⁻ effector cells relative to the wild-type IL-2. In one embodiment, the CD25⁻ cells are CD8⁺ effector T cells or NK cells. In one embodiment, in the STATS phosphorylation assay, the ability of the IL-2 mutant protein to activate CD25⁻ cells is identified by measuring the EC₅₀ value of the IL-2 mutant protein in activating STATS phosphorylation signals in CD25⁻ cells. In one embodiment, as determined in the STATS phosphorylation assay, the ability of the IL-2 mutant protein disclosed herein to activate CD25⁻ cells is enhanced by at least 1 times, e.g., 2 times, 3 times, 4 times, 5 times, or 10 times, relative to the wild-type IL-2 protein (e.g., human IL-2 set forth in SEQ ID NO: 1).

In one embodiment, the IL-2 mutant protein disclosed herein eliminates or reduces the preference of IL-2 for preferentially activating CD25⁺ cells relative to the wild-type IL-2. In one embodiment, the CD25⁺ cells are CD25⁺ CD8⁺ T cells. In another embodiment, the CD25⁺ cells are Treg cells. In one embodiment, in the STATS phosphorylation assay, the ability of the IL-2 mutant protein to activate CD25⁻ cells is identified by measuring the EC₅₀ values of the IL-2 mutant protein in activating STATS phosphorylation signals in CD25⁻ cells and in CD25⁺ cells, respectively. For example, the activation preference of the IL-2 mutant protein for CD25⁺ cells is determined by calculating the ratio of EC₅₀ values of the IL-2 mutant protein in activating STATS phosphorylation signals in CD25⁻ and in CD25⁺ T cells. Preferably, the preference of the mutant protein for CD25⁺ cells is reduced by at least 10 times, preferably at least 100 times, 150 times, 200 times, or 300 times or more, relative to the wild-type protein.

### The mutant protein disclosed herein

The IL-2 protein is a member of the short chain type I cytokine family with four α-helical bundles (A, B, C, and D). According to the analysis of the crystal structure (PDB: 1Z92), IL-2 has the following amino acid sites that interact with CD25 (i.e., IL-2Rα) in the region of amino acid residues 35-72: 35, 37, 38, 41, 42, 43, 45, 61, 62, 68, and 72. The inventors have found that by introducing specific mutations to the sites that interact with CD25 (i.e., sites 35, 37, 38, 41, 42, 43, 45, 61, 62, 68, and 72) in the region of amino acid residues 35-72 of IL-2, the binding of IL-2 to IL-2Rα can be reduced or eliminated. Herein, the mutations at these sites in this region are referred to as "CD25 binding region" mutations.

Moreover, by comparing the crystal structure of an IL-2 monomer (PDB: 1M47) with that of a complex (PDB: 2ERJ), the inventors have found that the B'C' loop was absent from the crystal structure of the IL-2 monomer since it was very active in a solution and could not form a relatively stable conformation. However, by genetically engineering the B'C' loop, for example, by sequence replacement or truncation, the stability of the B'C' loop can be increased, and thus the druggability of IL-2 and/or the binding affinity of IL-2 for the IL-2RP receptor can be improved. Herein, these sequence replacement or truncation mutations in the B'C' loop region are referred to as "B'C' loop region mutations".

The inventors have further found that the "CD25 binding region" mutations and the "B'C' loop region mutations" can be combined to further improve the properties of IL-2. Thus, the present invention provides an IL-2 mutant protein, which comprises: (i) "CD25 binding region" mutations; and (ii) "B'C' loop region mutations" compared to the wild-type IL-2 (preferably the human IL-2, and more preferably the IL-2 comprising the sequence of SEQ ID NO: 1).

### CD25 binding region mutation

In one aspect, the IL-2 mutant protein disclosed herein, relative to the wild-type IL-2, comprises one or more mutations in the CD25 binding region, preferably at positions 35, 37, 38, 41, 42, 43, 45, 61, 68 and 72. The mutation eliminates or reduces the binding affinity for the IL-2Rα receptor.

In some embodiments, the CD25 binding region mutations disclosed herein comprise a combination of mutations selected from one of the following combinations (1)-(9):

| Combinations | Mutations |
|---|---|
| 1 | K35D/E+T37E/D+R38Y/W+F42N/Q+Y45R/K+E61R/K+E68K/R, preferably K35D/E+T37E/D+R38W+F42Q+Y45R/K+E61R/K+E68K/R, and more preferably K35D/E+T37E/D+R38W+F42Q+Y45K+E61K+E68R |
| 2 | K35D/E+T37D/E+R38D/E+F42V/L/I/A, preferably K35D/E+T37D/E+R38D/E+F42V/A, and more preferably K35D/E+T37D/E+R38D/E+F42A |
| 3 | K35E/D+R38D/E+T41D/E+K43D/E, preferably K35E/D+R38D/E+T41E+K43E |
| 4 | K35E/D+T37D/E+R38E/D+K43F/Y+Y45R/K+L72Y/F, more preferably K35E/D+T37D/E+R38E/D+K43Y+Y45K+L72F |
| 5 | K35D/E+R38D/E+T41D/E+K43F/Y+Y45R/K+L72Y/F, more preferably K35D/E+R38D/E+ T41D/E+K43Y + Y 45K +L 72F |
| 6 | K35E/D+T37D/E+R38E/D+T41D/E+K43D/E+L72Y/F, more preferably K35E/D+T37D/E+R38E/D+T41D/E+K43D/E+L72F |
| 7 | K35E/D+T37D/E+R38E/D+K43D/E+L72Y/F, more preferably K35E/D+T37D/E+R38E/D+K43D/E+L72F |
| 8 | K35D/E+T37E/D+R38E/D+K43D/E+L72Y/F, more preferably K35D/E+T37E/D+R38E/D+K43D/E+L72F |
| 9 | K35D/E+R38E/D+T41D/E+K43D/E+E61R/K+L72Y/F, more preferably K35D/E+R38E/D+T41D/E+K43D/E+E61K+L72F |

In a preferred embodiment, the CD25 binding region mutations disclosed herein comprise a combination of mutations selected from one of the following combinations (1)-(9), particularly from one of the combinations (1)-(6):

| Combinations | Mutations |
|---|---|
| 1 | K35E+T37D+R38W+F42Q+Y45K+E61K+E68R |
| 2 | K35E+T37E+R38E+F42A |
| 3 | K35D+R38E+T41E+K43E |
| 4 | K35D+T37E+R38D+K43Y+Y45K+L72F |
| 5 | K35E+R38E+T41E+K43Y+Y45K+L72F |
| 6 | K35D+T37E+R38D+T41E+K43E+L72F |
| 7 | K35D+T37E+R38D+K43E+L72F |
| 8 | K35E+T37D+R38D+K43E+L72F |
| 9 | K35E+R38D+T41E+K43E+E61K+L72F |

In one preferred embodiment, the CD25 binding region mutations disclosed herein comprise or consist of the combination of mutations K35**E**+T37**E**+R38**E**+F42**A**.

In a preferred embodiment, the CD25 binding region mutations disclosed herein lead to eliminated CD25 binding, e.g., CD25 binding below the detection limit, as measured by a ForteBio affinity assay.

For CD25 binding region mutations suitable for the present invention, see also the applicant's co-pending application PCT/CN2019/107055, which is incorporated herein by reference in its entirety.

### B'C' loop region mutations

The IL-2 protein is a member of the short chain type I cytokine family with four α-helical bundles (A, B, C, and D). As used herein, the terms "B'C' loop", "B'C' loop region" and "B'C' loop sequence" are used interchangeably, referring to a linker sequence between the B and C helices of the IL-2 protein. The B'C' loop sequence of an IL-2 protein can be determined by performing analysis of the IL-2 crystal structure (e.g., PDB: 2ERJ). For the purpose of the present invention, according to the numbering of SEQ ID NO: 1, the B'C' loop sequence refers to a sequence linking the residue at position 72 to the residue at position 84 in the IL-2 polypeptide. In the wild-type IL-2 proteins set forth in SEQ ID NOs: 1, 2 and 3, the linker sequence comprises 11 amino acids, namely A73-R83.

As used herein, the term "shortened loop region" or "shortened B'C' loop region" means that a mutant protein has a B'C' loop sequence with a reduced length relative to the wild-type IL-2 protein, i.e., the linker sequence between the amino acid residues aa72 and aa84 is shortened according to the numbering of SEQ ID NO: 1. A "shortened loop region" can be achieved by replacement or truncation of the loop sequence. The replacement or truncation may occur in any region or portion of the B'C' loop sequence. For example, the replacement or truncation may be the replacement of the sequence A73-R83 in the loop region or the truncation of the sequence by one or more amino acid residues at the C-terminus. As another example, the replacement or truncation may be the replacement of the sequence A74-R83 in the loop region or the truncation of the sequence by one or more amino acid residues at the C-terminus. After the replacement or truncation, if necessary, a single amino acid substitution, e.g., an amino acid substitution for eliminating glycosylation and/or a reverse mutation, can be further introduced into the loop region sequence to further improve the performance of the mutant protein, e.g., the druggability. Therefore, herein, the mutated shortened B'C' loop region can be described through a sequence linking the residue at position 72 to the residue at position 84 after a mutation is introduced.

In one aspect, the IL-2 mutant protein disclosed herein, relative to the wild-type IL-2, comprises a B'C' loop region mutation; preferably, the mutation leads to a B'C' loop region with increased stability; more preferably, the mutation leads to improved druggability of the IL-2 mutant protein disclosed herein, e.g., an increased expression yield and/or purity.

In some embodiments, due to the mutation introduced, the mutant protein comprises a shortened B'C' loop region (i.e., a shortened linker sequence between the amino acid residues aa72 and aa84) compared to the wild-type IL-2 (preferably the human IL-2, and more preferably the IL-2 comprising the sequence of SEQ ID NO: 1), wherein, preferably, the shortened loop region has less than 10, 9, 8, 7, 6 or 5 amino acids in length, and more preferably has 7 amino acids in length; and the amino acid residues are numbered according to SEQ ID NO: 1.

Herein, B'C' loop region mutations suitable for the present invention include truncations and replacements of the B'C' loop region. In one embodiment, the mutations include truncations or replacements of the amino acid residues aa73 to aa83 in the B'C' loop region, e.g., a truncation to form A(Q/G)S(K/A/D)N(F/I)H, or a replacement with SGDASIH. In another embodiment, the mutations include truncations or replacements of the amino acid residues aa74 to aa83 in the B'C' loop region, e.g., a truncation to form (Q/G)S(K/A/D)N(F/I)H, or a replacement with GDASIH.

In some embodiments, the IL-2 mutant protein disclosed herein comprises a B'C' loop chimeric mutation. The mutant protein, relative to the wild-type IL-2, comprises a substitution of the entirety or a part of the sequence linking aa72 to aa84, for example, with a short B'C' loop sequence from other four-helical short-chain cytokine family members. The short B'C' loop suitable for the substitution of the wild-type IL-2 can be identified from other four-helical short-chain cytokine IL family members, such as IL-15, IL-4, IL-21, or IL family members from non-human species such as mice, by the superpose of a crystal structure. In one embodiment, the sequence used for substitution is a B'C' loop sequence from interleukin IL-15, particularly human IL-15. In one embodiment, the substitution includes substitutions of the amino acid residues aa73 to aa83 in the B'C' loop region. In another embodiment, the substitution includes substitutions of the amino acid residues aa74 to aa83 in the B'C' loop region. Preferably, the IL-2 mutant protein disclosed herein, after the substitutions, has a B 'C' loop sequence (i.e., a sequence linking aa72 to aa84) selected from the following: SGDASIH and AGDASIH.

In some embodiments, the IL-2 mutant protein disclosed herein comprises a B'C' loop truncation mutation. The mutant protein, relative to the wild-type IL-2, comprises a truncation of the sequence linking aa72 to aa84. In one embodiment, the truncation includes truncations of the amino acid residues aa73 to aa83 in the B'C' loop region. In another embodiment, the truncation includes truncations of the amino acid residues aa74 to aa83 in the B'C' loop region. For example, the sequence may be truncated by 1, 2, 3 or 4 amino acids at the C-terminus. Preferably, after the truncation, the IL-2 mutant protein disclosed herein has a B'C' loop region having a sequence of A(Q/G)S(K/A/D)N(F/I)H. Preferably, after the truncation, the IL-2 mutant protein disclosed herein has a B'C' loop sequence (i.e., a sequence linking aa72 to aa84) selected from the following:

| B'C' loop sequence |
|---|
| AQSKNFH |
| AQSANFH |
| AQSDNFH |
| AGSKNFH |
| AQSANFH |
| AQSANIH |

In one preferred embodiment, the IL-2 mutant protein disclosed herein comprises a B'C' loop region sequence selected from the following: AQSKNFH, AQSANFH, AQSDNFH, SGDASIH and AGDASIH.

For B'C' loop mutations suitable for the present invention, see also the applicant's co-pending application PCT/CN2019/107054, which is incorporated herein by reference in its entirety.

### Preferred exemplary combinations of mutations

In some preferred embodiments, the B'C' loop mutations and the CD25 binding region mutations disclosed herein are combined to provide two or all three of the following improved properties: (i) reduced (or eliminated) binding to IL-2Rα, (ii) enhanced binding to IL-2Rα, and (ii) improved expression level and purity.

In some embodiments, the present invention provides an IL-2 mutant protein, which comprises, relative to the wild-type IL-2:
(i) a combination of mutations selected from one of the following combinations (1)-(9), particularly from one of the combinations (1)-(6):

| Combinations | Mutations |
|---|---|
| 1 | K35**E**+T37**D**+R38**W**+F42**Q**+Y45**K**+E61**K**+E68**R** |
| 2 | K35**E**+T37**E**+R38**E**+F42**A** |
| 3 | K35**D**+R38**E**+T41**E**+K43**E** |
| 4 | K35**D**+T37**E**+R38**D**+K43**Y**+Y45**K**+L72**F** |
| 5 | K35**E**+R38**E**+T41**E**+K43**Y**+Y45**K**+L72**F** |
| 6 | K35**D**+T37**E**+R38**D**+T41**E**+K43**E**+L72**F** |
| 7 | K35**D**+T37**E**+R38**D**+K43**E**+L72**F** |
| 8 | K35**E**+T37**D**+R38**D**+K43**E**+L72**F** |
| 9 | K35**E**+R38**D**+T41**E**+K43**E**+E61**K**+L72**F** |

and (ii) a B'C' loop region sequence selected from the following:

| B'C' loop sequence |
|---|
| SGDASIH |
| AGDASIH |
| AQSKNFH |
| AQSANFH |
| AQSDNFH |
| AGSKNFH |
| AQSANFH |
| AQSANIH |

particularly from the following B'C' loop region sequences: AQSKNFH, AQSANFH AQSDNFH, SGDASIH and AGDASIH.

Preferably, the present invention provides an IL-2 mutant protein, which comprises, relative to the wild-type IL-2,
(i) a combination of mutations K35**E**+T37**E**+R38**E**+F42**A**; and
(ii) a B'C' loop region sequence selected from AQSKNFH, AQSANFH, AQSDNFH, SGDASIH and AGDASIH.

Therefore, in one embodiment, the present invention provides an IL-2 mutant protein that has a mature region having at least 85% or 90% identity in an amino acid sequence to that of the wild-type IL-2 protein set forth in one of SEQ ID NOs: 1-3, and also comprises a linker sequence between amino acid positions 72 and 84 selected from AGDASIH, SGDASIH, AQSKNFH, AQSANFH, AQSDNFH, AGSKNFH, AQSANFH and AQSANIH, and has a combination of mutations: K35**E**+T37**E**+R38**E**+F42**A**.

In some preferred embodiments, the present invention provides an IL-2 mutant protein that comprises an amino acid sequence that has at least 90%, 92%, 93%, 94%, 95%, 96%, 97% or 98% identity to an amino acid sequence selected from one of SEQ ID NOs: 22, 23, 24, 25 and 26. In some embodiments, the mutant protein comprises or consists of the amino acid sequences of SEQ ID NOs: 22, 23, 24, 25 and 26.

Preferably, due to the combinatorial mutation, the IL-2 has a reduced preference for preferentially stimulating p-STATA5 signaling in CD25⁺ T cells and an enhanced ability to stimulate signaling in CD25⁻ T cells.

### Other mutations

In addition to the mutations in the above "CD45 binding" region and "B'C' loop region", the IL-2 mutant protein disclosed herein can also have one or more mutations in other regions or positions, as long as it retains one or more beneficial properties described above. For example, the IL-2 mutant protein disclosed herein may also comprise a substitution at position 125, such as C125S, C125A, C125T, or C125V, so as to provide additional advantages, such as improved expression or homogeneity or stability (see, e.g., U.S. Patent No. 4,518,584). As another example, the IL-2 mutant protein disclosed herein can also comprise a substitution at position 3, e.g., T3A, to remove the O-glycosylation at the N-terminus of IL2. As another example, the IL-2 mutant protein disclosed herein can also comprise a substitution at position 76, e.g., K76D/A, to enhance the T cell activation activity. Those skilled in the art know how to determine additional mutations that can be incorporated into the IL-2 mutant protein disclosed herein.

The sequence difference between the IL-2 mutant protein and the wild-type protein can be expressed in terms of sequence identity or in terms of the number of different amino acids between the two. In one embodiment, the IL-2 mutant protein has at least 85%, 86%, 87%, 88%, or 89% identity, preferably more than 90% (preferably 95%) but preferably no more than 97%, and more preferably no more than 96% identity to the wild-type protein. In another embodiment, in addition to the above CD25 region mutations and B'C' loop region mutations described herein, the IL-2 mutant protein may also have no more than 15, e.g., 1-10, or 1-5, e.g., 0, 1, 2, 3 or 4, mutations relative to the wild-type protein. In one embodiment, the additional mutations may be conservative substitutions. In one embodiment, the additional mutations occur outside of the CD25 region and the B'C' loop region.

### 2. Fusion Protein and Immunoconjugate

The present invention also provides a fusion protein comprising the IL-2 mutant protein disclosed herein. In one preferred embodiment, the IL-2 mutant protein disclosed herein is fused to another polypeptide, such as albumin, and preferably an antibody Fc fragment, which can provide improved pharmacokinetic properties. In one embodiment, the Fc fragment comprises a mutation that reduces or removes effector functions, such as the L234A/L235A mutation or L234A/L235E/G237A mutation that reduces binding to an Fcy receptor. Preferably, the Fc-containing fusion protein has an increased serum half-life. In one preferred embodiment, the Fc-containing fusion protein also has reduced Fc-mediated effector functions, such as reduced or eliminated ADCC or ADCP or CDC effector functions.

In some embodiments, the Fc fragment fused to the IL-2 mutant protein is a human IgG Fc, e.g., human IgG1 Fc, human IgG2 Fc or human IgG4 Fc. In one embodiment, the Fc fragment comprises the amino acid sequence of SEQ ID NO: 7, or has at least 90% identity, e.g., 95%, 96%, 97%, 99% or higher identity, thereto.

In some embodiments, the IL-2 mutant protein is fused to the Fc via a linker. In some embodiments, the linker may be selected to enhance the activation of the Fc fusion protein on CD25⁻ T cells. In one embodiment, the linker is GSGS, preferably 2 × (G4S).

In some embodiments, the Fc fusion protein comprises an amino acid sequence having at least 85%, at least 95%, or at least 96% identity to an amino acid sequence selected from SEQ ID NOs: 15-19. In some embodiments, the Fc fusion protein consists of the sequences of SEQ ID NOs: 15-19.

The present invention also provides an immunoconjugate comprising the IL2 mutant protein disclosed herein and an antigen-binding molecule. Preferably, the antigen-binding molecule is an immunoglobulin molecule, particularly an IgG molecule, an antibody, or an antibody fragment, and more particularly an Fab molecule or an scFv molecule. In some embodiments, the antigen-binding molecule specifically binds to an antigen present on a tumor cell or in tumor environment, such as an antigen selected from: fibroblast activation protein (FAP), A1 domain of tenascin-C (TNC A1), A2 domain of tenascin-C (TNC A2), extra domain B (EDB) of fibronectin, carcinoembryonic antigen (CEA), and melanoma-associated chondroitin sulfate proteoglycan (MCSP). Thus, the immunoconjugate disclosed herein can target the tumor cell or the tumor environment after being administrated to a subject, thereby providing further therapeutic benefits, such as the feasibility of treatment at lower doses and the consequent low side effects, and enhanced anti-tumor effects.

In the fusion protein and immunoconjugate disclosed herein, the IL-2 mutant protein disclosed herein can be linked, either directly or through a linker, to another molecule or antigen-binding molecule, and in some embodiments, a proteolytic cleavage site is provided therebetween.

### 3. Polynucleotide, Vector, and Host

The present invention provides a nucleic acid encoding any of the IL-2 mutant proteins, fusions or conjugates above. The polynucleotide sequence encoding the mutant protein disclosed herein can be generated by *de novo* solid phase DNA synthesis or by PCR mutagenesis of an existing sequence encoding the wild-type IL-2 using methods well known in the art. In addition, the polynucleotide and the nucleic acid disclosed herein may comprise a segment encoding a secretion signal peptide and are operably linked to a segment encoding the mutant protein disclosed herein so that secretory expression of the mutant protein disclosed herein can be directed.

The present invention also provides a vector comprising the nucleic acid disclosed herein. In one embodiment, the vector is an expression vector, such as a eukaryotic expression vector. The vector includes, but is not limited to, a virus, a plasmid, a cosmid, a λ phage, or a yeast artificial chromosome (YAC). In a preferred embodiment, the expression vector disclosed herein is a pCDNA3.1 expression vector.

In addition, the present invention also provides a host cell comprising the nucleic acid or the vector. Host cells suitable for replicating and supporting the expression of the IL-2 mutant protein, the fusion or the immunoconjugate are well known in the art. Such cells can be transfected or transduced with a particular expression vector, and a large number of cells comprising vectors can be cultivated for inoculation in large-scale fermenters, so as to obtain sufficient IL-2 mutants, fusions or immunoconjugates for clinical application. In one embodiment, the host cell is eukaryotic. In another embodiment, the host cell is selected from a yeast cell and a mammalian cell (e.g., a CHO cell or a 293 cell). For example, the polypeptide may be produced in a bacterium, particularly when glycosylation is not required. After expression, the polypeptide can be isolated from the bacterial cell paste in a soluble fraction and can be further purified. In addition to prokaryotes, eukaryotic microorganisms such as filamentous fungi or yeast are cloning or expression hosts for the vector suitable for encoding the polypeptide, including fungal and yeast strains in which the glycosylation pathway has been "humanized", which results in the production of the polypeptide with a partially or fully human glycosylation pattern. See Gerngross, NatBiotech, 22,1409-1414 (2004) and Li et al., NatBiotech, 24,210-215 (2006). Examples of available mammalian host cell lines include SV40 transformed monkey kidney CV1 lines (COS-7), human embryonic kidney lines (293 or 293T cells, as described, for example, in Graham et al., JGenVirol 36,59 (1977)), baby hamster kidney cells (BHK), mouse Sertoli cells (TM4 cells, as described, for example, in Mather, BiolReprod 23,243-251 (1980)), monkey kidney cells (CV1), African green monkey kidney cells (VERO-76), human cervical cancer cells (HELA), canine kidney cells (MDCK), buffalo rat liver cells (BRL3A), human lung cells (W138), human liver cells (HepG2), mouse mammary tumor cells (MMT060562), TRI cells (as described, for example, in Mather et al., AnnalsN.Y.AcαdSci 383,44-68 (1982)), MRC5 cells, and FS4 cells. Other available mammalian host cell lines include Chinese hamster ovary (CHO) cells, including dhfr-CHO cells (Urlaub et al., Proc. Natl. Acad. Sci. USA 77,4216 (1980)), and myeloma cell lines such as YO, NS0, P3X63, and Sp2/0. In one embodiment, the host cell is a eukaryotic cell, preferably a mammalian cell such as a Chinese hamster ovary (CHO) cell, a human embryonic kidney (HEK) cell, or a lymphocyte (e.g., Y0, NS0, and Sp20 cells).

### 4. Preparation Method

In a further aspect, the present invention provides a method for preparing the IL-2 mutant protein, the fusion or the conjugate disclosed herein, wherein the method comprises culturing a host cell comprising a nucleic acid encoding the protein, the fusion or the conjugate under conditions suitable for expression of the IL-2 mutant protein, the fusion or the conjugate, as provided above, and optionally isolating the protein, the fusion or the conjugate from the host cell (or the host cell culture medium).

### 5. Assay

The IL-2 mutant protein provided herein can be identified, screened, or characterized for its physical/chemical properties and/or biological activities through a variety of assays known in the art.

In one aspect, the IL-2 mutant protein disclosed herein can be tested for its binding activity to an IL-2 receptor.

For example, the binding to a human IL-2Rα or β protein can be determined by methods known in the art, such as ELISA, and Western blotting, or by the exemplary methods disclosed in the examples herein. For example, the flow cytometry can be used, wherein cells such as yeast display cells that are transfected to express the mutant protein on the cell surface react with a labeled (e.g., biotin-labeled) IL-2Rα or β protein. Alternatively, the binding of the mutant protein to the receptor, including the binding kinetics (e.g., the K_{D} value), can be determined by a ForteBio assay using a recombinant mutant protein-Fc fusion.

In a further aspect, the ability of the IL-2 mutant protein to bind to the IL-2 receptor can be measured indirectly by measuring the signaling and/or immune activation at the downstream of receptor binding.

Thus, in some embodiments, an assay for identifying the IL-2 mutant protein having a biological activity is provided. The biological activities may include, for example, the ability to induce proliferation of T cells and/or NK cells and/or Treg cells with IL-2 receptors, the ability to induce IL-2 signaling in T cells and/or NK cells and/or Treg cells with IL-2 receptors, reduced ability to induce apoptosis in T cells, the ability to induce tumor regression and/or to improve survival, and reduced *in vivo* toxicity properties, such as reduced vascular permeability. The present invention also provides an IL-2 mutant protein having such biological activities *in vivo* and/or *in vitro.*

Various methods known in the art can be used for determining the biological activities of the IL-2. For example, an assay suitable for testing the ability of the IL-2 mutant protein disclosed herein to stimulate IFN-γ production by NK cells may comprise the steps of: incubating the cultured NK cells with the IL-2 mutant protein, the fusion or the immunoconjugate disclosed herein, and measuring the IFN-γ concentration in the culture medium by ELISA. IL-2 signaling induces several signaling pathways and involves JAK (Janus kinase) and STAT (signal transducers and activators of transcription) signaling molecules.

The interaction of the IL-2 with the β and γ subunits of the receptor results in phosphorylation of the receptor and JAK1 and JAK3 (which bind to the β and γ subunits, respectively). STATS then binds to the phosphorylated receptor and is phosphorylated on a very important tyrosine residue. This results in dissociation of STATS from the receptor, dimerization of STATS, and translocation of STATS dimers to the nucleus where they facilitate the transcription of target genes. Thus, the ability of the mutant IL-2 polypeptide to induce signaling via the IL-2 receptor can be assessed, for example, by measuring the phosphorylation of STATS. Details of this method have been disclosed in the examples. For example, PBMCs can be treated with the mutant IL-2 polypeptide, the fusion or the immunoconjugate disclosed herein, and the level of phosphorylated STATS is determined by flow cytometry.

Furthermore, the effect of the mutant IL-2 on tumor growth and survival can be assessed in a variety of animal tumor models known in the art. For example, heterografts of cancer cell lines can be implanted into immunodeficient mice and treated with the mutant IL-2 polypeptide, the fusion or the immunoconjugate disclosed herein. The *in vivo* anti-tumor effects of the mutant IL-2 polypeptide, the fusion and the immunoconjugate disclosed herein can be determined based on tumor growth inhibition (e.g., calculated relative to an isotype control antibody). In addition, the *in vivo* toxicity of the mutant IL-2 polypeptide, the fusion and the immunoconjugate disclosed herein can be determined based on changes in the body weight of animals (e.g., changes in the absolute body weight or percent changes in the body weight relative to the body weight before administration). The *in vivo* toxicity can also be determined based on mortality, life-time observations (visible symptoms of adverse effects, e.g., behavior, body weight, and body temperature), and clinical and anatomical pathology (e.g., measurement of blood chemistry values and/or histopathological analysis).

In a further aspect, the druggability (e.g., expression yield and product purity) of the mutant protein disclosed herein can be characterized by using methods known in the art. For the determination of the expression yield, when the mutant protein is secreted from the cultured cells and expressed in the culture supernatant, the cell culture fluid collected by centrifugation can be assayed for the protein content. Alternatively, the assay may be performed after one-step purification of the collected cell culture fluid, for example, after one-step affinity chromatography purification. For the determination of the purity of the product, the purity can be determined after one-step affinity chromatography purification of the collected culture supernatant of the production cells to determine the purification performance of the mutant protein. Preferably, the mutant protein disclosed herein, after being purified by this one-step affinity chromatography, has significantly higher purity than the wild-type protein, indicating that the mutant protein disclosed herein has a better purification performance. The purity determination method can be any conventional method known in the art, including, but not limited to, the SEC-HPLC method.

In a further aspect, the storage stability of the IL-2 mutant proteins disclosed herein can also be determined by using methods known in the art. In the present invention, a "stable" antibody means that the antibody formulated in a buffer retains an acceptable degree of physical and/or chemical stability after being stored under specific conditions. In one embodiment, the buffer is PBS buffer at pH 7.4. In another embodiment, the buffer is a histidine buffer at pH 6.5. In one embodiment, the antibody is tested for stability after being stored at 40 °C for a period of time, e.g., 2 weeks or longer. The stability of the antibody can be determined by measuring the reduction in the purity of the stored antibody by using the SEC-HPLC method or the CE-SDS method.

### 6. Screening Method

In a further aspect, the present invention provides a method for obtaining an IL-2 mutant protein with improved properties and the IL-2 mutant protein obtained by using this method.

In one embodiment, the method disclosed herein comprises the following steps:
(1) introducing one or more mutations by performing mutation at the binding interface of IL-2 to IL-2Rα, and shortening the sequence of the B'C' loop region of the IL-2 by mutation therein,
   preferably introducing the combination of the CD25 binding region mutations described above and/or the chemeric or truncation mutations in the B'C' loop sequence described above; and
(2) expressing the IL-2 mutant protein in a mammalian cell (e.g., an HEK293 or CHO cell), for example, in the form of an Fc fusion (e.g., an FcLALA fusion);
   - identifying a mutant protein having one or more of the following improved properties: (i) improved expression yield and/or protein purity after purification (e.g., purity as measured by SEC-HPLC after one-step affinity chromatography); (ii) reduced binding to IL2Rα; and (iii) enhanced binding to IL2Rβ.

In one embodiment, the method comprises: identifying the IL-2 mutation that improves druggability (e.g., expression yield and/or product stability and/or homogeneity, such as one-step Fc affinity chromatography purity) before the combination of mutations in step (1) is performed. In one preferred embodiment, the druggability of the mutant protein is improved by substituting the B'C' loop with a shortened B'C' loop or truncating the B'C' loop to form a shortened B'C' loop.

In one embodiment, the method comprises: identifying the IL-2 mutation that imparts a reduced ability to bind to IL-2Rα relative to the wild-type IL-2 before the combination of mutations in step (1) is performed. In one embodiment, the CD25 binding affinity is reduced or eliminated by performing amino acid substitutions or combined substitutions at sites in the CD25 binding region.

As will be appreciated by those skilled in the art, these mutations can be combined with mutations imparting further improved druggability or other improved properties to obtain an IL-2 mutant protein with multiple improved properties.

In one embodiment, the combination of a CD25 region mutation that results in reduced binding to IL2Rα (e.g., known) and a mutation that truncates and/or substitutes the B'C' loop region to improve the druggability (e.g., known) is introduced into the IL-2 protein, and the properties are identified. In one preferred embodiment, the identification includes reduced binding to IL-2Rα and improved druggability (e.g., improved expression and/or purity, and/or product stability and/or homogeneity), and optionally substantially unchanged or weakened or enhanced binding affinity for IL-2RP, relative to the wild-type IL-2.

In some embodiments, the parental wild-type IL-2 protein used as a mutation template preferably has at least 85%, or at least 90% or 95% identity to SEQ ID NO: 1, and more preferably is an IL-2 protein derived from human.

### 7. Pharmaceutical Composition and Pharmaceutical Preparation

The present invention also comprises a composition (including a pharmaceutical composition or a pharmaceutical preparation) comprising the IL-2 mutant protein or the fusion or immunoconjugate thereof, and a composition comprising the polynucleotide encoding the IL-2 mutant protein or the fusion or immunoconjugate thereof. Such compositions can further optionally comprise suitable pharmaceutical adjuvants, such as a pharmaceutical carrier and a pharmaceutical excipient known in the art, including buffers.

The pharmaceutical carrier applicable to the present invention may be sterile liquid, such as water and oil, including those derived from petroleum, animals, plants or synthesis, such as peanut oil, soybean oil, mineral oil, and sesame oil. Water is a preferred carrier when the pharmaceutical composition is administered intravenously. Saline solutions, aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol, etc. For use and application of excipients, see Handbook of Pharmaceutical Excipients, 5th Ed., R. C. Rowe, P. J. Seskey and S. C. Owen, Pharmaceutical Press, London, Chicago. The composition may further comprise a small quantity of wetting agent, emulsifier, or pH buffer, if desired. The compositions may be in the form of a solution, a suspension, an emulsion, a tablet, a pill, a capsule, a powder, a sustained release preparation, and the like. Oral preparations may comprise standard carriers, such as pharmaceutical grade mannitol, lactose, starch, magnesium stearate, and saccharin.

The pharmaceutical preparation comprising the IL-2 mutant protein can be formulated by mixing the IL-2 mutant protein, the fusion or the immunoconjugate disclosed herein of a desired purity with one or more optional pharmaceutical excipients (Remington's Pharmaceutical Sciences, 16th edition, Osol, A. eds. (1980)), preferably in the form of a lyophilized preparation or an aqueous solution. An exemplary lyophilized antibody preparation is described in U.S. Patent No. 6,267,958. The aqueous antibody preparation includes those described in U.S. Patent No. 6,171,586 and WO2006/044908, and the latter preparation comprises a histidine-acetate buffer. In addition, a sustained release preparation can be prepared. Suitable examples of the sustained release preparation include a semipermeable matrix of a solid hydrophobic polymer comprising a protein. The matrix is in the form of a shaped article, such as a film or a microcapsule.

In one embodiment, the pharmaceutical composition disclosed herein comprises a buffer at pH 6-8, e.g., PBS buffer or histidine buffer. In one embodiment, the PBS buffer is, e.g., a PBS buffer at about pH 7.4. In one embodiment, the histidine buffer is a histidine buffer at about pH 6.5, e.g., comprising 10 mM histidine, 5% sorbitol, and 0.02% polysorbate 80. The pharmaceutical composition disclosed herein is preferably stable when being stored in the buffer.

The pharmaceutical composition or preparation disclosed herein can further comprise one or more other active ingredients which are required for a specific indication being treated, preferably active ingredients having complementary activities that do not adversely affect one another. For example, it is desirable to further provide other anti-cancer active ingredients, such as chemotherapeutic agents and immune checkpoint inhibitors. The active ingredients are suitably combined in an amount effective for an intended purpose.

### 8. Combination Product

In one aspect, the present invention further provides a combination product comprising the mutant protein or the fusion or immunoconjugate thereof disclosed herein, and one or more other therapeutic agents (e.g., a chemotherapeutic agent, other antibodies, a cytotoxic agent, a vaccine, and an anti-infective active agent). The combination product disclosed herein can be used in a therapeutic method disclosed herein.

In some embodiments, the present invention provides a combination product, wherein the aforementioned other therapeutic agents refer to, for example, a therapeutic agent, such as an antibody, which is effective to stimulate an immune response and thus further enhance, stimulate or upregulate the immune response in a subject.

In some embodiments, the combination product is used for preventing or treating cancer. In some embodiments, the cancer is, e.g., a gastrointestinal cancer, e.g., rectal cancer, colon cancer, or colorectal cancer. In some embodiments, the combination product is used for preventing or treating an infection, such as bacterial infection, viral infection, fungal infection, and protozoal infection.

### 9. Therapeutic Method and Use

As used herein, the terms "individual" and "subject" are used interchangeably and refer to a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In particular, a subject is a human.

As used herein, the term "treating" refers to a clinical intervention intending to alter the natural progress of a disease in an individual being treated. Desired therapeutic effects include, but are not limited to, preventing the occurrence or recurrence of diseases, alleviating symptoms, reducing any direct or indirect pathological outcomes of diseases, preventing metastasis, delaying disease progression, improving or alleviating conditions, and alleviating or improving prognosis.

In one aspect, the present invention provides a method for stimulating the immune system of a subject, comprising administering to the subject an effective amount of a pharmaceutical composition comprising the IL-2 mutant protein, the fusion or the immunoconjugate disclosed herein. The IL-2 mutant protein disclosed herein has high activity and selectivity for CD25⁻ CD122⁺ effector cells (cytotoxic CD8⁺ T cells and NK cells), and has a reduced stimulation effect on CD25⁺ Treg cells. The IL-2 mutant protein disclosed herein can be used at a low dose to stimulate the immune system of the subject.

Thus, in some embodiments, the present invention relates to a method for enhancing the immune response of the body of a subject, comprising administering to the subject an effective amount of any of the IL-2 mutant proteins or the fusions or immunoconjugates thereof described herein. In some embodiments, the IL-2 mutant protein or the fusion or immunoconjugate thereof disclosed herein is administered to a subject with a tumor to stimulate an anti-tumor immune response. In other embodiments, the antibodies or the antigen-binding fragments thereof disclosed herein are administered to a subject with an infection to stimulate an anti-infection immune response.

In another aspect, the present invention relates to a method for treating a disease, such as cancer, in a subject, wherein the method comprises administering to the subject an effective amount of any of the IL-2 mutant proteins or the fusions or immunoconjugates thereof described herein. The cancer may be at an early, intermediate or advanced stage, or may be a metastatic cancer. In some embodiments, the cancer may be, e.g., a gastrointestinal cancer, e.g., rectal cancer, colon cancer, or colorectal cancer.

In another aspect, the present invention relates to a method for treating an infectious disease, e.g., chronic infection, in a subject, wherein the method comprises administering to the subject an effective amount of any of the IL-2 mutant proteins or the fragments thereof, or an immunoconjugate, a multispecific antibody, or a pharmaceutical composition comprising the antibodies or the fragments described herein. In one embodiment, the infection is virus infection.

The mutant protein disclosed herein (or the pharmaceutical composition comprising the same, or the fusion or immunoconjugate thereof, and optionally an additional therapeutic agent) can be administered by any suitable method, including parenteral administration, intrapulmonary administration, intranasal administration, and, if required by locoregional treatment, intralesional administration. Parenteral infusion includes intramuscular, intravenous, intra-arterial, intraperitoneal or subcutaneous administration. The administration is carried out by any suitable means, such as injection, e.g., intravenous or subcutaneous injection, to some extent depending on whether the treatment is short-term or long-term. Various administration schedules are encompassed herein, including, but not limited to, single administration or multiple administrations at multiple time points, bolus injection, and pulse infusion.

In order to prevent or treat a disease, the appropriate dosage of the mutant protein disclosed herein (used alone or in combination with one or more additional therapeutic agents) will depend on the type of the disease to be treated, the type of the antibody, severity and progression of the disease, the purpose for which the antibody is administered (prevention or treatment), previous treatments, clinical history of a patient, responses to the antibody, and the discretion of an attending physician. The antibody is suitably administered to a patient through a single treatment or through a series of treatments.

In a further aspect, the present invention also provides use of the IL-2 mutant protein, composition, immunoconjugate, and fusion disclosed herein in preparation of a drug for use in the aforementioned method (e.g., for treatment).

The following examples are described to assist in understanding the present invention. The examples are not intended to be and should not be interpreted in any way as limiting the protection scope of the present invention.

### Examples

### Example 1: Design and Construction of Interleukin-2 Point Mutant Library

### Design of an interleukin-2 point mutant library

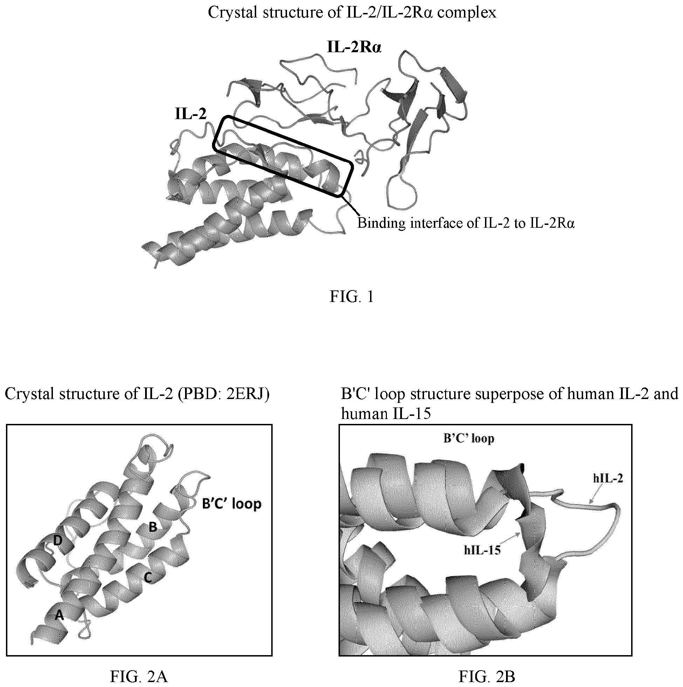

According to the crystal structure (PDB: 1Z92) (as shown in FIG. 1) of the complex of interleukin-2 (referred to as IL-2) and its alpha receptor CD25 (referred to as IL-2Rα), the listed IL-2 residues at interaction sites were mutated as per Table 1. The original amino acids at each site accounted for 50%, and the remaining 50% was divided equally by the "mutant amino acids" in Table 1. The theoretical diversity of the library designed for the binding site of IL-2 to IL-2Rα was 3×8×8×9×6×6×3×6×6×5×6 ≈ 2.0 × 10⁸, and the library was named as IBYDL029 (Innoventbio Yeast Display Library).

**Table 1. Mutation sites for the IBYDL029 library**

| Sites | Amino acid residues | Mutant amino acids | Diversity |
|---|---|---|---|
| 35 | Lys(K) | D,E | 3 |
| 37 | Thr(T) | D,E,R,K,F,Y,W | 8 |
| 38 | Arg(R) | D,E,F,Y,W,A,V | 8 |
| 41 | Thr(T) | K,R,M,F,Y,W,Q,E | 9 |
| 42 | Phe(F) | K,R,A,E,Q | 6 |
| 43 | Lys(K) | E,D,F,Y,W | 6 |
| 45 | Tyr(Y) | R,K | 3 |
| 61 | Glu(E) | R,K,W,Y,L | 6 |
| 62 | Glu(E) | R,K,W,Y,L | 6 |
| 68 | Glu(E) | R,K,W,Y | 5 |
| 72 | Leu(L) | R,K,F,Y,W | 6 |

### Construction of an interleukin-2 mutant library

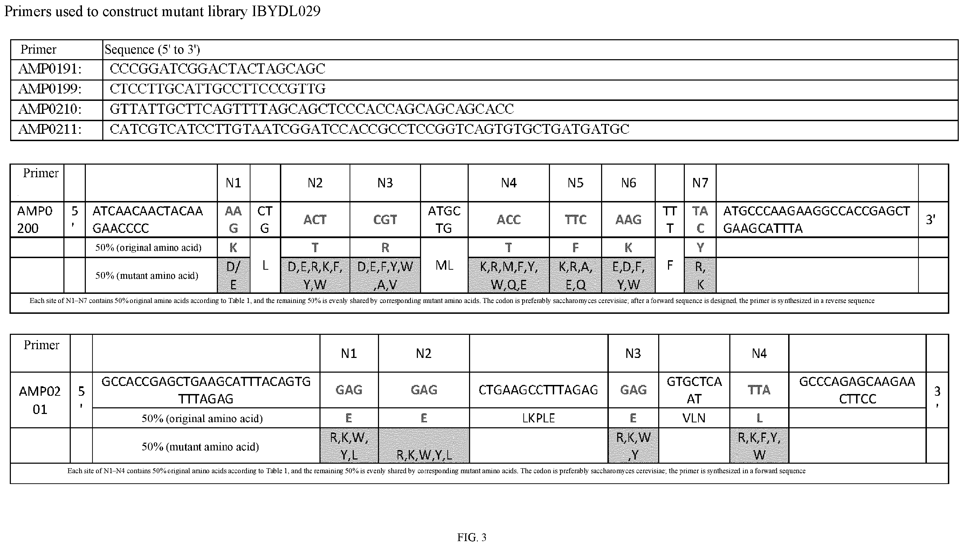

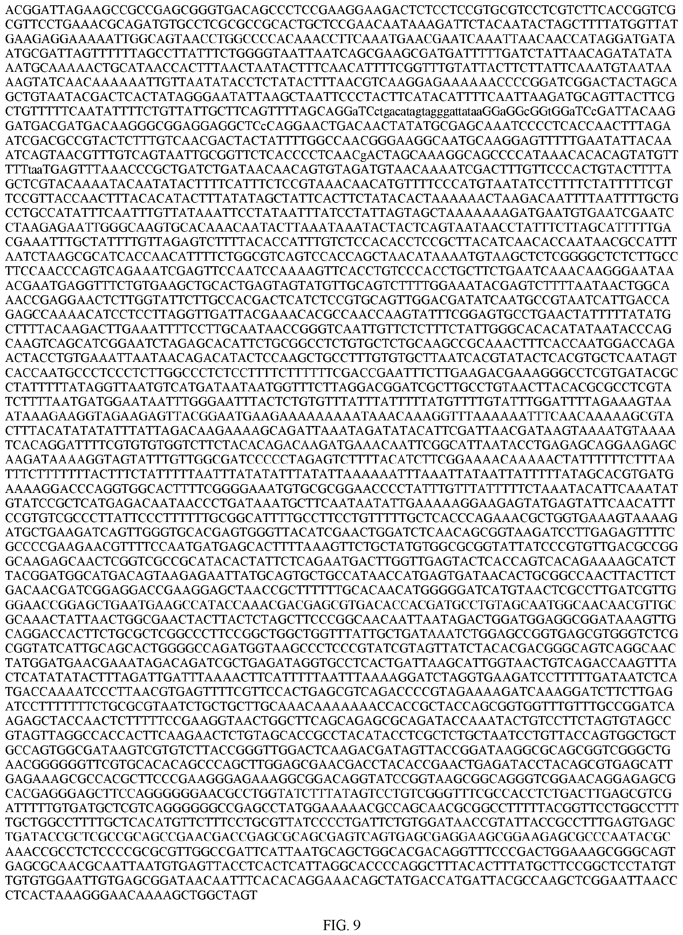

The wild-type IL-2 (UniProt: P60568, aa21-153, C125S, referred to as IL-2^{WT}) was placed between the two BamHI restriction enzyme cutting sites of yeast surface display plasmid pYDC011 (see FIG. 9 for the complete sequence of the plasmid). The sequence of IL-2^{WT} was set forth in SEQ ID NO: 1 in the present application. A C125S mutation was introduced at position 125 of the sequence to avoid the formation of a disulfide-bridged IL-2 dimer. The specific steps of plasmid construction were as follows:
1. fragments were amplified using primers AMP0210 and AMP0211, with IL-2^{WT} gene as a template (see FIG. 3 for the primer sequence);
2. plasmid pYDC011 was digested with BamHI (New England Biolab, Catalog No. R3136V), followed by gel extraction (QIAGEN Gel Extraction Kit, Catalog No. 28704);
3. the amplified product and digested product were extracted from 1% agarose gel;
4. after extraction, the products were homologously recombined *in vitro* using One Step Cloning Kit (Vazyme Catalog No. C113-02);
5. the recombined product was transferred into Escherichia coli Top10 competent cells (Tiangen, Catalog No. CB104-02), and the cells were coated on an ampicillin-resistant LB plate and cultured at 37 °C overnight;
6. after the growing monoclonal colonies were verified by sequencing, the correct plasmid was named as pYDC035.

According to existing literature, the IL-2 mutant IL-2^{3X} does not bind to IL-2Rα, and has unchanged binding to IL-2RP (Rodrigo Vazquez-Lombardi et al., Nature Communications, 8:15373, DOI: 10.1038/ncomms15373). IL-2^{3X} was displayed on the surface of yeasts and used as a control. The sequence of IL-2^{3X} was set forth in SEQ ID NO: 4. Similar to IL-2^{WT}, the protein also comprised a C125S mutation.

The required primers (as shown in FIG. 3) were designed according to the scheme for library construction in Table 1 and synthesized by Suzhou Genewiz Biological Technology Co., Ltd.

IBYDL029 library DNA amplification: 1. fragment 029-F was amplified with primers AMP0191 and AMP0200 using pYDC035 as a template; 2. fragment 029-R was amplified with primers AMP0201 and AMP0199 using pYDC035 as a template; 3. fragments 029-F and 029-R were extracted from gel and used as a PCR amplification template to amplify the full-length fragment 029 with primers AMP0191 and AMP0199.

100 µg of plasmid pYDC011 was digested with BamHI, and extracted using a PCR product recovery kit (QIAGEN PCR Purification Kit, Catalog No. 28104) to give a sufficient amount of linear plasmid. The linear plasmid and the library DNA were mixed at a ratio of 4 µg:12 µg. The mixture of the library DNA and the linear plasmid was electrotransfected into the EBY100 yeast strain according to the existing method (Lorenzo Benatuil et al., An improved yeast transformation method for the generation of very large human antibody libraries. Protein Engineering, Design & Selection vol. 23, no. 4, pp. 155-159, 2010). After electrotransfection, the library was serially diluted and applied to an SD-Trp (TAKARA, Catalog No. 630309) plate. The number of growing colonies was counted. The actual diversity of the library obtained was: IBYDL029: 4.2 × 10⁸, which was greater than the theoretical diversity of the library.

### Example 2: Screening and Identification of IL-2 Point Mutant Library

### Preparation of IL-2Rα and IL-2Rβ proteins and biotin labeling

### Construction and transfection of expression plasmids

An avi tag (GLNDIFEAQKIEWHE, the tag peptide can be biotinylated under BirA enzyme catalysis) and 6 histidine tags (HHHHHH) were linked to the C-terminus of the sequences of the IL-2 receptors IL-2Rα (UniProt: P01589, aa22-217) and IL-2RP (UniProt: P14784, aa27-240), which were constructed separately into pTT5 vectors (Addgene) to express IL-2Rα and IL-2RP proteins. The sequences of the receptors constructed were set forth in SEQ ID NOs: 5 and 6.

The cultured HEK293-F (Invitrogen, Catalog No. R79007) cells were transiently transfected with the expression plasmid vectors constructed above using a chemical transfection reagent polyethyleneimine (referred to as PEI, Polysciences, Catalog No. 23966) according to a scheme provided by the manufacturer.

### Expression and purification of proteins

The culture fluids of the cells expressing IL-2Rα and IL-2RP proteins were centrifuged at 4500 rpm for 30 min, and the cells were discarded. The supernatants were filtered through a 0.22 µm filter and further purified. Briefly, a nickel column (5 mL Histrap excel, GE, 17-3712-06) used for purification was soaked with 0.1 M NaOH for 2 h, and then washed with 5-10 column volumes of ultra-pure water to remove alkali liquor. The column was equilibrated with 5 column volumes of binding buffer (20 mM Tris pH 7.4, 300 mM NaCl) prior to purification. The cell supernatant was passed through the equilibrated column, and the column was washed with 10 column volumes of wash buffer (20 mM Tris pH 7.4, 300 mM NaCl, 10 mM imidazole) to remove non-specific binding impurity proteins. The target protein was then eluted with 3-5 column volumes of eluent (20 mM Tris pH 7.4, 300 mM NaCl, 100 mM imidazole). The collected protein was buffer-exchanged into PBS (Gibco, 70011-044) by ultrafiltration concentration, and further separated and purified using superdex200 increase (GE, 10/300GL, 10245605). The elution peak of the monomer was collected, and the equilibration buffer and elution buffer for the column were PBS (Gibco, 70011-044). 100 µg of the purified protein sample was taken and the protein purity was determined using a gel filtration column SW3000 (TOSOH Catalog No. 18675).

### Biotin labeling of IL-2Rα and IL-2Rβ proteins

The IL-2Rα and IL-2RP proteins were labeled with biotin by enzymatic method, of which the procedures were as follows: an appropriate amount of solutions of the IL-2Rα and IL-2RP proteins expressed and purified above were added with 1/10 (m/m) mass of His-BirA protein (UniProt: P06709), followed by ATP (Sigma, Catalog No. A2383-10G) with a final concentration of 2 mM, MgCl₂ with a final concentration of 5 mM, and D-biotin (AVIDITY, Catalog No. K0717) with a final concentration of 0.5 mM; the mixtures were incubated at 30 °C for 1 h, and purified by Superdex200 increase (GE, 10/300GL, 10245605) to remove excess biotin and His-BirA; the purified samples were verified by Streptavidin (SA) sensor (PALL, 18-5019) from Fortebio to confirm the successful biotin labeling. The biotin-labeled IL-2Rα and IL-2RP IH proteins obtained in this example were referred to as IL-2Rα-Biotin and IL-2RP IH-Biotin, respectively.

### Screening of IL-2 mutant library to give IL-2^{mutant} and differential staining

### Screening for IL-2^{mutant} that do not bind to IL-2Rα but bind to IL-2Rβ

From the yeast-based IL-2^{mutant} display library IBYDL029 with a diversity of 2.0 × 10⁸, 2.0 × 10⁹ yeast cells were taken for culture and induction. Due to the large diversity of the IBYDL029 library, magnetic-activated cell sorting was performed using the MACS system from Miltenyi in the first round of screening. First, 2 × 10⁹ yeast cells were incubated in FACS washing buffer (1× PBS, containing 1% bovine serum albumin) for 30 min at room temperature, and the buffer contained 500 nM biotin-labeled IL-2RP (Aero Biosystems, labeled with EZ-Link Sulfo-NHS-LC-Biotin, referred to as IL-2Rβ-Biotin). The cells were washed once with 50 mL of pre-cooled FACS buffer and resuspended in 10 mL of the same buffer, followed by addition of 40 µL of streptavidin microbeads (Miltenyi biotec, Catalog No. 130-090-485) and incubation at 4 °C for 15 min. The mixture was centrifuged at 3000 rpm for 3 min. After discarding the supernatant, the cells were resuspended in 10 mL of FACS buffer. The resulting cell suspension was loaded on a Miltenyi LS column. After loading, the column was washed three times, each with 3 mL of FACS buffer. The Miltenyi LS column was removed from the magnetic field and eluted with 5 mL of growth medium. The eluted yeast cells were collected and incubated overnight at 30 °C.

The cells of the library obtained through the first round of screening were induced by shaking at 20 °C for 24 h to display IL-2^{mutant}, and the second round of sorting was performed using flow cytometer. Briefly, 3 × 10⁷ yeast cells taken from the library were washed three times with FACS buffer, added to FACS buffer containing IL-2Rβ-Biotin (300 nM) and Anti Flag antibody, and incubated at room temperature for 30 min. After being washed twice with FACS washing buffer, the cells were mixed with FACS washing buffer containing SA-PE (phycoerythrin-labeled streptavidin, eBioscience, Catalog No. 12-4317-87) and goat anti-mouse IgG conjugated with Alex Flour-647 (Thermo Fisher, Catalog No. A21235), and incubated in the dark at 4 °C for 15 min. The cells were washed twice with pre-cooled FACS washing buffer, resuspended in 1 mL of buffer, and transferred into a separator tube with a filter. The cells were sorted using MoFlo_XDP, and the sorted yeast cells were incubated overnight at 30 °C. The sorting scheme of the third round was the same as that of the second round. After three rounds of screening, the monoclones were picked and sent for sequencing.

After three rounds of screening using IL-2Rβ-Biotin, 53 mutant sequences were obtained from the library IBYDL029.

### Differential staining of IL-2^{mutant}

Yeast cells comprising a single mutant sequence after sequencing were induced by shaking at 20 °C for 24 h to display IL-2^{mutant}, and stained together with their receptors IL-2Rα-Biotin and IL-2RP IH-Biotin, respectively. The specific steps were as follows:
I. staining analysis of IL-2^{mutant} displayed yeast cells together with IL-2Rα-Biotin:
   1. 1 × 10⁶ cells from each sample were centrifuged to discard the supernatant, and washed with FACS buffer once for later use;
   2. the cells were added to 100 µL of FACS buffer containing 50 nM IL-2Rα-Biotin and Anti Flag antibody, and incubated at room temperature for 30 min;
   3. the mixture was centrifuged at 3000 rpm at 4 °C for 3 min, and washed twice with pre-cooled FACS buffer;
   4. 100 µL of FACS buffer containing SA-PE and goat anti-mouse conjugated with Alex Flour-647 was added, and the mixture was incubated in the dark on ice for 20 min;
   5. after being washed twice with pre-cooled FACS buffer, the cells were resuspended in 100 µL of buffer, and the binding level of IL-2^{mutant} to IL-2Rα was assayed by a flow cytometer (BD,
   ACCURI C6).
II. Staining analysis of IL-2^{mutant} displayed yeast cells together with IL-2Rβ IH-Biotin:
   1. 1 × 10⁶ cells from each sample were centrifuged to discard the supernatant, and washed with FACS buffer once for later use;
   2. the cells were added to 100 µL of FACS buffer containing IL-2Rβ IH-Biotin (30 nM-100 nM) and Anti Flag antibody, and incubated at room temperature for 30 min;
   3. following steps 3-5 described above, the binding level of IL-2^{mutant} to IL-2RP was assayed.

It can be seen from the staining results of flow cytometry that: the mean fluorescence intensity of binding of 53 IL-2^{mutant} obtained through the screening of the library IBYDL029 to IL-2Rα was close to that of IL-2^{3X}, i.e., neither of them bound to IL-2Rα; and the mean fluorescence intensity of binding to IL-2RP was stronger than that of IL-2^{3X}.

### Expression of IL-2^{mutant}-FC fusion proteins and determination of their affinity (avidity) for receptors Construction of expression plasmids

The IL-2^{mutant} sequence was linked to FcLALA via two GGGGS and constructed into a vector of pCDNA3.1 (Addgene) to express the IL-2^{mutant}-FC fusion protein.

In addition, as controls, IL-2^{WT} and IL-2^{3X} gene sequences were linked to FcLALA via two GGGGS and constructed into pCDNA3.1 to express the IL-2^{WT}-FC and IL-2^{3X}-FC fusion proteins. The Fc used in this and subsequent examples was the Fc of human IgG1 with the mutations L234A and L235A (referred to as FcLALA, SEQ ID NO: 7).

### Expression and purification of fusion proteins of IL-2 and FC

A vector containing the gene encoding the fusion protein described above was transferred into HEK293 cells using a chemical transfection method. The cultured HEK293 cells were transiently transfected using chemical transfection reagent PEI according to a scheme provided by the manufacturer. First, the plasmid DNA and the transfection reagent were prepared in a superclean bench. 3 mL of Opti-MEM medium (Gibco, Catalog No. 31985-070) was added to a 50 mL centrifuge tube, followed by 30 µg of the corresponding plasmid DNA. The Opti-MEM medium containing the plasmid was filtered with a 0.22 µm filter, and then added with 90 µg of PEI (1 g/L), and the mixture was let stand for 20 min. The DNA/PEI mixture was gently poured into 27 mL of HEK293 cells, mixed well, and cultured at 37 °C with 8% CO₂ for 20 h, followed by the addition of VPA to reach a final concentration of 2 mM. Then 2% (v/v) of Feed was added, and the resulting mixture was cultured for another 6 days.

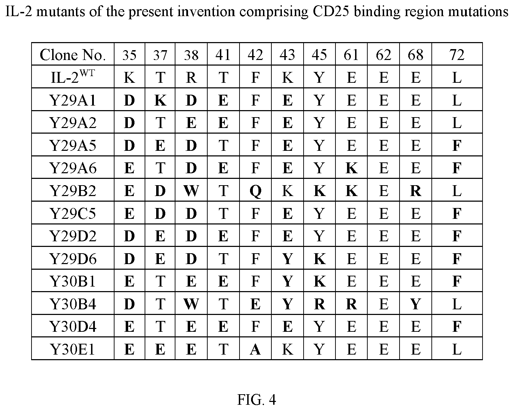

After culture, the mixture was centrifuged at 13000 rpm for 20 min, and the supernatant was collected, and purified by pre-packed column Hitrap Mabselect Sure. The procedures were as follows: the packing column was equilibrated with 5 column volumes of equilibration buffer (20 mM Tris, 150 mM NaCl, pH 7.2) before purification; the collected supernatant was passed through the column, and then the column was washed with 10 column volumes of equilibration buffer to remove non-specific binding proteins; the packing was washed with 5 column volumes of elution buffer (1 M sodium citrate, pH 3.5), and the eluent was collected; 80 µL of Tris (2 M Tris) was added per 1 mL of eluent, and the mixture was buffer-exchanged into PBS using an ultrafiltration concentration tube, and then the concentration was determined. 100 µg of purified protein was taken with its concentration adjusted to 1 mg/mL. The purity of IL-2-Fc dimer protein was determined by gel filtration chromatography. The results are shown in Table 2. The sequences of the IL-2 mutants disclosed herein listed in Table 2 are shown in FIG. 4.

**Table 2. Expression yield and purity of IL-2-FC in 293 cells**

| IL-2 | Expression yield (mg/L) | Purity (SEC-HPLC) |
|---|---|---|
| IL-2^{WT} | 18 | 45% |
| Mutant IL-2^{3X} | 34 | 70% |
| Mutant Y29A1 | No expression | N.D |
| Mutant Y29A2 | 37 | 79% |
| Mutant Y29A5 | 12 | 73% |
| Mutant Y29A6 | 1 | 37% |
| Mutant Y29B2 | 77 | 88% |
| Mutant Y29C5 | 10 | 60% |
| Mutant Y29D2 | 17 | 80% |
| Mutant Y29D6 | 23 | 60% |
| Mutant Y30B1 | 20 | 70% |
| Mutant Y30B4 | No expression | N.D |
| Mutant Y30D4 | No expression | N.D |
| Mutant Y30E1 | 60 | 78% |

### Determination of affinity of IL-2^{mutant}-FC fusion proteins for their receptors

The equilibrium dissociation constants (K_{D}) for binding of the IL-2^{mutant}-FC fusion proteins disclosed herein to their receptors were measured by biological optical interferometry (ForteBio).

The ForteBio affinity assay was conducted according to the method (Estep, P et al., High throughput solution Based measurement of antibody-antigen affinity and epitope binding. mAbs, 2013.5(2): p. 270-8) known in the art. Briefly, the affinity of candidate IL-2^{mutant}-FC for each of IL-2Rα and IL-2RP was measured as follows: a sensor was equilibrated offline in an assay buffer for 20 min and equilibrated online for 120 s to establish a baseline; the human biotin-labeled IL-2Rα or IL-2RP was loaded onto an SA sensor (PALL, 18-5019) for ForteBio affinity assay; the sensor loaded with IL-2Rα-Biotin or IL-2RP IH-Biotin was placed into a solution containing 100 nM IL-2^{mutant}-FC until to a plateau, and then transferred to an assay buffer for dissociation for at least 2 min to measure the association and dissociation rates. The kinetic analysis was performed using a 1:1 binding model.

In the assay described above, the affinity K_{D} values of IL-2^{mutant}-FC fusion proteins expressed by HEK293-F cells for their receptors are shown in Table 3. As a control, the affinity K_{D} values of the IL-2^{WT}-FC and IL-2^{3X}-FC fusion proteins measured using the same method are also shown in Table 3.

**Table 3. Affinity K_{D} values of IL-2^{mutant}-FC fusion proteins for their receptors**

| | | |
|---|---|---|
| IL-2 | IL-2Rα avidity | IL-2RP avidity |
| IL-2^{WT} | 1.0E-08 | 4.1E-08 |
| IL-2^{3X} | N.B | 1.6E-08 |
| Y29A2 | Very weak | 4.8E-10 |
| Y29A5 | N.B | 3.4E-10 |
| Y29A6 | N.B | 1.8E-09 |
| Y29B2 | N.B | 3.4E-10 |
| Y29C5 | N.B | 4.2E-10 |
| Y29D2 | Very weak | 8.5E-11 |
| Y29D6 | N.B | 1.9E-10 |
| Y30B1 | N.B | 1.9E-10 |
| Y30E1 | N.B | 5.9E-09 |

| | | |
|---|---|---|
| N.B: none binding | | |

It can be seen from the affinity data, the binding of all of the above mutants obtained from the library IBYDL029 to IL-2Rα was blocked while the binding to IL-2RP was maintained.

### Example 3: Construction, Screening and Identification of IL-2 Chimeric and Truncated Mutants

### Design of an IL-2 B'C' loop chimera and an IL-2 B'C' loop truncate

B'C' loop: The linker sequence (FIG. 2A) of the B helix and C helix of the IL-2, comprising 11 amino acids, namely A73-R83.

By comparing the crystal structure of an IL-2 monomer (PDB: 1M47) with that of a complex (PDB: 2ERJ), it was found that the B'C' loop was absent from the crystal structure of the IL-2 monomer since it was very active in a solution and could not form a relatively stable conformation.

By genetically engineering the B'C' loop, the stability of the B'C' loop, and thus the stability of the IL-2 and the affinity of the IL-2 for the IL-2RP, was increased. We therefore observed the crystal structure of the human IL-15 (PDB: 2Z3Q) and found that its B'C' loop was relatively short and stable (FIG. 2B). Therefore, we designed an IL-2 chimeric molecule (L017) and 4 truncated molecules (L057-L060) (see Table 4).

**Table 4. Optimized sequences of IL-2 B'C' loop**

| Name | B'C' loop sequence |
|---|---|
| L 001(IL-2^{WT}) | AQSKNFHLRPR |
| L 017(IL-2^{hyb15BCL}) | SGDASIH |
| L 057(IL-2^{truncate1}) | AQSKNFH |
| L 058(IL-2^{truncate2}) | AGSKNFH |
| L 059(IL-2 ^{truncate3}) | AQSANFH |
| L 060(IL-2 ^{truncate4}) | AQSANIH |

### Construction of expression plasmids

The wild-type IL-2 (UniProt: P60568, aa21-153, C125S, IL-2^{WT} for short), the IL-2 mutant IL-2^{3X} (R38D, K43E, E61R), and the B'C' loop chimeras and truncates were linked to the Fc of human IgG1 (L234A, L235A, FcLALA for short, SEQ ID NO: 7) via a GSGS linker sequence and constructed into pTT5 vectors to express the following proteins:

| Protein | Structure | SEQ ID NOs |
|---|---|---|
| Y001 | IL-2^{WT}-GSGS-FcLALA | SEQ ID NO: 8 |
| Y002 | IL-2^{.3X}-GSGS-FcLALA | SEQ ID NO: 9 |
| Y017 | IL-2^{hyb15BCL}-GSGS-FcLALA | SEQ ID NO: 10 |
| Y057 | IL-2^{truncate1}-GSGS-FcLALA | SEQ ID NO: 11 |
| Y058 | IL-2^{truncate2}-GSGS-FcLALA | SEQ ID NO: 12 |
| Y059 | IL-2 ^{truncate3}-GSGS-FcLALA | SEQ ID NO: 13 |
| Y060 | IL-2 ^{truncate4}-GSGS-FcLALA | SEQ ID NO: 14 |

The B'C' loop chimera (Y017) or truncate (Y057), combined with the mutant Y30E1 (K35E, T37E, R38E, F42A) obtained by library screening, was linked to FcLALA via two GGGGS and constructed into pCDNA3.1 vectors to express the following proteins. Among them, Y092 had a chimeric B'C' loop sequence AGDASIH, and the potential N-glycosylation resulting from the amino acid residues NLS at position 71-73 in Y017 was removed; Y093 and Y094 were obtained by introducing a further amino acid substitution K76A or K76D into the truncated loop sequence on the basis of Y089, which was intended to improve the T cell activation activity of the B 'C' loop truncate; Y144 had an additional T3A on the basis of Y092, which was intended to remove the O-glycosylation at the N-terminus of IL2.

| Protein | Structure | SEQ ID NOs |
|---|---|---|
| Y089 | IL-2^{.Y30E1.truncate1}-2^{∗}(G4S)-FcLALA | SEQ ID NO: 15 |
| Y092 | IL-2^{. Y30E1.15BCL}-2^{∗}(G4S)-FcLALA | SEQ ID NO: 16 |
| Y093 | IL-2^{.Y30E1.truncate1.K76A}-2^{∗}(G4S)-FcLALA | SEQ ID NO: 17 |
| Y094 | IL-2^{.Y30E1.truncate1.K76D}-2^{∗}(G4S)-FcLALA | SEQ ID NO: 18 |
| Y144 | IL-2^{. Y30E1.15BCL.T3A} -2^{∗}(G4S)-FcLALA | SEQ ID NO: 19 |

Moreover, the IL-2^{WT} and the IL-2^{3X} were linked to FcLALA via two GGGGS and constructed into pCDNA3.1 vectors to express the following proteins:

| Protein | Structure | SEQ ID NOs |
|---|---|---|
| Y040 | IL-2^{.3X}-2^{∗}(G4S)-FcLALA | SEQ ID NO: 20 |
| Y045 | IL-2^{WT}-2*(G4S)-FcLALA | SEQ ID NO: 21 |

The specific sequence information of the above protein molecules is shown in the sequence listing.

### Expression and purification of IL-2 fusion proteins

The above protein molecules were expressed in 293 cells and CHO cells, respectively. The expression in HEK293 cells was performed by using the method for expressing IL-2-Fc fusion proteins in Example 2. The expression in CHO cells was performed as follows.

ExpiCHO cells (Invitrogen) were passaged according to a desired cell volume. The cell density was adjusted to 3.5 × 10⁶ cells/mL the day before transfection. The cell density (about 8-10 × 10⁶ cells/mL) was measured on the day of transfection. The viability reached 95% or more. The cell density was adjusted to 6 × 10⁶ cells/mL using ExpiCHO^{™} Expression Medium (Gibco, Catalog No. A29100-01). OptiPRO^{™} SFM (Gibco, Catalog No. 12309-019) at a final volume of 8% (v/v) was taken as a transfection buffer. A corresponding amount (0.8 µg/mL cell) of plasmid was added, and the mixture was well mixed and filtered through a 0.22 µm filter membrane to remove the bacteria. The reagent in ExpiFectamine^{™} CHO Transfection Kit (Gibco, Catalog No. A29130) was added in a cell ratio of 3.2 µL/mL. The complex formed with transfection reagent and the plasmid DNA was incubated at room temperature for 1-5 min, and slowly added to the cells. The cells were cultured at 37 °C with 8% CO₂ for 18 h, and then 0.6% (v/v) Enhancer and 30% (v/v) Feed were added. The cells were cultured for another 6 days.

After culture, the cell culture fluid was centrifuged at 13000 rpm for 20 min, and the supernatant was collected and purified by a pre-packed column Hitrap Mabselect Sure (GE, 11-0034-95). The procedures were as follows: the packing column was equilibrated with 5 column volumes of equilibration buffer (20 mM Tris, 150 mM NaCl, pH 7.2) before purification; the collected supernatant was passed through the column, and then the column was washed with 10 column volumes of equilibration buffer to remove non-specific binding proteins; the packing was washed with 5 column volumes of elution buffer (100 mM sodium citrate, pH 3.5), and the eluent was collected. 80 µL of Tris (2 M Tris) was added per 1 mL of eluent, and the mixture was buffer-exchanged into PBS (Gibco, Catalog No. 70011-044) using an ultrafiltration concentration tube (MILLIPORE, Catalog No. UFC901096), and then the concentration was determined. 100 µg of purified protein was taken with its concentration adjusted to 1 mg/mL. The protein purity was determined by gel filtration column SW3000 (TOSOH Catalog No. 18675).

The fusion proteins of the B'C' loop chimera (Y017) and truncate (Y057/058/059) had greatly improved expression yields and one-step affinity chromatography purity in HEK293 cells compared to the wild-type IL-2 fusion protein (Y001). The results are shown below in Table 5.

**Table 5. Expression yield and purity of IL-2 mutants in HEK293**

| Protein | Expression yield (mg/L) | Purity (SEC-HPLC) |
|---|---|---|
| Y001 | 16.35 | 44.74% |
| Y002 | 23.92 | 69.85% |
| Y017 | 54.47 | 93.45% |
| Y057 | 52.36 | 92.77% |
| Y058 | 49.86 | 99.09% |
| Y059 | 36.52 | 86.95% |
| Y060 | 21.20 | 66.33% |

The fusion proteins of the B'C' loop chimeras (Y092/144), truncates (Y089/093/094) and further mutant Y30E1 also had greatly improved expression yields and one-step affinity chromatography purity in CHO cells compared to the wild-type IL-2 fusion protein (Y045). The results are shown below in Table 6.

**Table 6. Expression yield and purity of IL-2 mutants in CHO**

| Protein | Expression yield (mg/L) | Purity (SEC-HPLC) |
|---|---|---|
| Y040 | 20.28 | 40.75% |
| Y045 | 2.44 | 50.85% |
| Y089 | 249.6 | 99.11% |
| Y092 | 118.8 | 99.07% |
| Y093 | 95.2 | 98.98% |
| Y094 | 142 | 99.02% |
| Y144 | 114 | 97.78% |

### Determination of the affinity of IL-2 mutant Fc fusion proteins for their receptors

The following mutant proteins were subjected to affinity K_{D} value determination according to the ForteBio affinity determination method described in Example 2. The results are shown below in Table 7.

**Table 7. K_{D} values of IL-2 mutants for IL-2Rα and IL-2RP**

| IL-2^{mutant} | IL-2Rα avidity | IL-2Rβ avidity |
|---|---|---|
| Y30E1 | N.B. | 6.45E-09 |
| Y089 | N.B. | 4.53E-09 |
| Y093 | N.B. | 1.96E-09 |
| Y094 | N.B. | 3.35E-09 |
| Y092 | N.B. | 3.27E-09 |
| Y144 | N.B. | 1.55E-09 |

| | | |
|---|---|---|
| N.B: none binding | | |

According to the above data, it can be seen that: 1) the point mutation molecules such as Y30E1 obtained by yeast library screening can block the binding to IL-2Rα; 2) the B'C' loop chimeric molecule and truncate molecule improved the molecule expression yield and purity and also the affinity of the molecule for IL-2Rβ; 3) the combination molecules of Y30E1 and the B'C' loop mutant achieved the blocking of IL-2Rα, improved molecule expression yield and purity, and also improved binding activity to IL-2Rβ.

### Example 4: In Vitro Functional Assays of IL-2 Mutants

Due to higher affinity for IL-2Rα than IL-2RP and IL-2Rγ, IL-2^{WT} will preferentially bind to IL-2Rα on the cell surface, then recruit IL-2Rβγ. The downstream p-STAT5 signals are released by IL-2Rβγ to stimulate the proliferation of T cells and NK cells. As the IL-2Rα is present on the surface of Treg cells but absent on the surface of effector T cells and NK cells, normally the IL-2^{WT} will preferentially stimulate the Treg cell proliferation and down regulate the immune response. Since the IL-2^{mutant} does not bind to the IL-2Rα, the preference of the IL-2^{mutant} for preferentially stimulating Treg cell proliferation is eliminated and meanwhile the number of effector T cells and NK cells is effectively increased by stimulating T cells and NK cells proliferation, thus improving the anti-tumor effect.

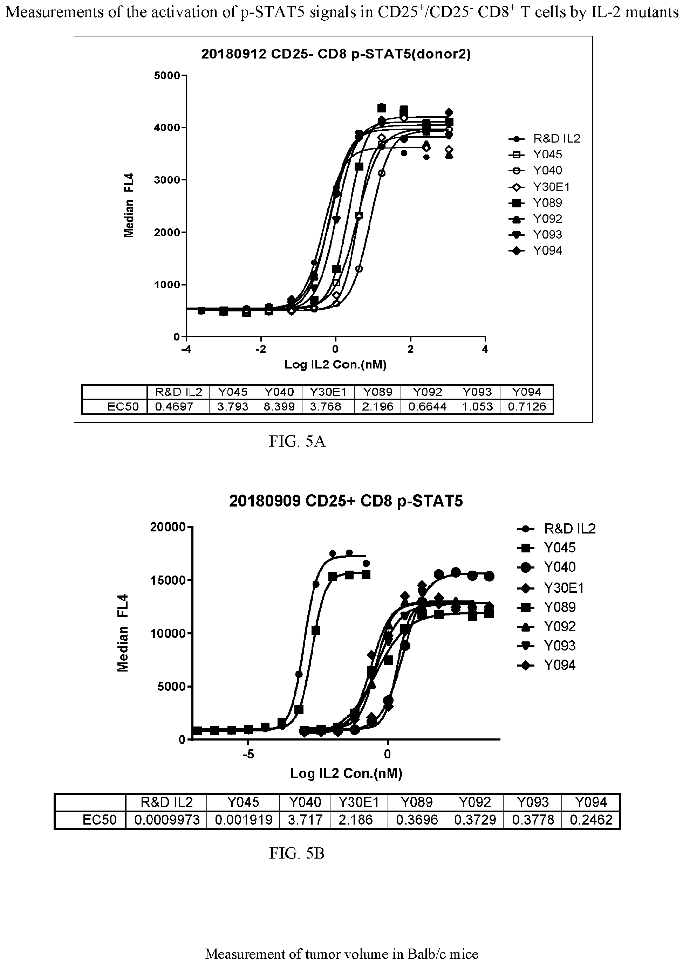

In this example, the elimination of the activation preference of each mutant for CD25⁺ cells was verified by detecting the activation effect of each IL-2^{mutant}-FC on p-STAT5 signals of primary human CD8⁺ T cells, and the mutant with a high activation effect on CD25⁻ cells was screened. The specific steps are as follows:
1. Thawing PBMC cells:
   a) PBMC cells (Allcells, Catalog No. PB005F, 100M package) were taken out from liquid nitrogen, and then rapidly placed in a 37 °C water bath for thawing;
   b) the cells were added to 10 mL of preheated X-VIVO15 (Lonza, Catalog No. 04-418Q) culture medium containing 5% human AB serum (GemCell, Catalog No. 100-512) and 1 ‰ DNase (STRMCELL, Catalog No. 07900), centrifuged at 400 G and 25 °C for 10 min (the subsequent centrifugation was under the same condition) and washed once;
   c) 20 mL of culture medium was added to resuspend the cells, and the cells were cultured overnight in a 37 °C carbon dioxide incubator.
2. Purifying human CD8⁺ T cells:
   a) the cell suspension obtained in step 1 was pipetted, and after centrifugation, the supernatant was discarded;
   b) a mixture of 1 mL of Robosep buffer (STEMCELL, Catalog No. 20104), 100 µL of human AB serum, and 100 µL of negative screening antibody in human CD8⁺ T cell purification kit (Invitrogen, Catalog No. 11348D) was added to resuspend the cells;
   c) after mixing well, the cells were incubated for 20 min at 4 °C and shaken every 5 min;
   d) after incubation, 10 mL of Robosep buffer was added, and the cells were centrifuged and washed twice;
   e) meanwhile, 1 mL of magnetic microspheres (human CD8⁺ T cell purification kit) was taken, and 7 mL of Robosep buffer was added; the mixture was placed on a magnetic frame for 1 min to discard the supernatant, and the magnetic microspheres were pre-washed;
   f) the microspheres and the cells were resuspended with 1 mL of Robosep buffer, and after mixing well, the mixture was subjected to rotary incubation for 30 min at room temperature;
   g) after incubation, 6 mL of Robosep buffer was added and the mixture was placed on a magnetic frame for 1 min, followed by the collection of the supernatant;
   h) the collected liquid was placed on the magnetic frame for 1 min, and the supernatant was collected;
   i) centrifugation was performed to discard the supernatant, the cells were resuspended using a preheated T culture medium, and the cell density was adjusted to 1 × 10⁶/mL;
   j) 1/3 of the cells were taken to stimulate the expression of CD25 later, and the remaining cells were placed in a 37 °C carbon dioxide incubator for static culture overnight.
3. Stimulating CD8⁺ T cells to express CD25:
   a) 1/3 of the CD8⁺ T cells purified in step 2 were taken, into which magnetic microspheres of an anti-human CD3/CD28 antibody (GIBCO, Catalog No. 11131D) were added (the ratio of cells to microspheres was 3:1);
   b) the mixture was placed in a 37 °C carbon dioxide incubator for static culture for three days;
   c) 10 mL of culture medium was added to wash the cells twice;
   d) the culture medium was added to adjust the cell density to 1 × 10⁶/mL, and the cells were placed in a 37 °C carbon dioxide incubator for static culture for 2 days.
4. Detecting the purity and expression level of the cells:
   a) an anti-human CD8-PE antibody (Invitrogen, Catalog No. 12-0086-42), an anti-human CD25-PE antibody (eBioscience, Catalog No. 12-0259-42), and an isotype control antibody (BD, Catalog No. 556653) were adopted to detect CD8 and CD25 of the cells;
   b) the cells in step 2 were CD8⁺ CD25⁻ T cells, and the cells in step 3 were CD8⁺ CD25⁺ T cells.
5. Detecting the EC₅₀ value of each IL-2^{mutant}-FC in activating p-STAT5 signals in CD8⁺ CD25⁻ T cells:
   a) CD8⁺ CD25⁻ T cells were added to 96-well U-bottom plates (Costar, Catalog No. CLS3799-50EA) at 1 × 10⁵ cells per well;
   b) the IL-2^{mutant}-FC, the commercialized IL-2 (R&D, Catalog No. 202-IL-500), the IL-2^{WT}-FC, and the IL-2^{3X}-FC, each of 100 µL, were added and 4-fold diluted in gradient from a maximum concentration of 266.7 nM, for a total of 12 dilution gradients, and the cells were incubated in a 37 °C incubator for 20 min;
   c) 55.5 µL of 4.2% formaldehyde solution was added to immobilize the above cells at room temperature for 10 min;
   d) centrifugation was performed to discard the supernatant, and 200 µL of ice methanol (Fisher, Catalog No. A452-4) was added to resuspend the cells, which were then incubated in a 4 °C refrigerator for 30 min;
   e) centrifugation was performed to discard the supernatant, and the residue was washed 3 times with 200 µL of staining buffer (BD, Catalog No. 554657);
   f) 200 µL of permeabilization/fixation buffer (BD, Catalog No. 51-2091KZ) containing anti-p-STAT5-AlexFlour647 (BD, Catalog No. 562076, 1:200 dilution) was added, and the cells were incubated in the dark for 3 h at room temperature;
   g) the cells were washed with staining buffer for three times, resuspended with 100 µL of staining buffer, and detected using a flow cytometer;
   h) the EC₅₀ values to activate p-STAT5 signals were plotted using the IL-2 molecule concentration as the abscissa and the AlexFlour647 median fluorescence value as the ordinate, and the results are shown in FIG. 5A and Table 8.
6. Detecting the EC₅₀ value of each IL-2^{mutant}-FC in activating p-STAT5 signals in CD8⁺ CD25⁺ T cells:
   a) CD8⁺ CD25⁺ T cells were added to 96-well U-bottom plates at 1 × 10⁵ cells per well;
   b) the EC₅₀ values to activate p-STAT5 signals were plotted through steps same as b-h in step 5, and the results are shown in FIG. 5B and Table 8.

**Table 8. EC50 of IL-2 mutants activating p-STAT5 signals in CD25^{+/-} T cells and ratios thereof**

| | R&D IL2 | Y045 | Y040 | Y30E1 | Y089 | Y092 | Y093 | Y094 |
|---|---|---|---|---|---|---|---|---|
| CD25-pSTAT5 EC₅₀ | 0.4697 | 3.793 | 8.399 | 3.768 | 2.196 | 0.6644 | 1.053 | 0.7126 |
| CD25+pSTAT5 EC₅₀ | 0.0009973 | 0.001919 | 3.717 | 2.186 | 0.3696 | 0.3729 | 0.3778 | 0.2462 |
| Ratio of CD25⁻ EC₅₀/CD25⁺ EC₅₀ | 470.9716 | 1976.5503 | 2.2596 | 1.7237 | 5.9416 | 1.7817 | 2.7872 | 2.8944 |

The experimental results show that: 1) all of the B'C' loop mutants of Y30E1, Y089, Y092, Y093 and Y094, activates p-STAT5 signals in CD25⁻ CD8⁺ T cells with lower EC₅₀ than Y30E1, indicating that the activation of CD25⁻ CD8⁺ T cells by the molecule can be improved after B'C' loop optimization, which is consistent with the affinity data for IL-2R[3; 2) according to the ratios of CD25⁻ EC₅₀/CD25⁺ EC₅₀, the mutant proteins Y30E1, Y089, Y092, Y093 and Y094 have significantly reduced preference for the activation of CD25⁺ cells relative to Y045 (IL-2WT-Fc), indicating that the Y30E1 mutation safely blocked the binding to CD25.

### Example 5: Stability of IL-2 Mutants

The stability of Y089, Y092 and Y094 stored in a PBS buffer (pH 7.4, Gibco, Catalog No.10010-023) or a histidine buffer (10 mM histidine, 5% sorbitol, 0.02% polysorbate 80, adjusted with hydrochloric acid to pH 6.5) in a 40 °C thermostatic incubator (Zhicheng SHP-150) for 7 days and 14 days was evaluated. The test indexes were the purities of IL-2 mutant proteins determined by SEC-HPLC and CE-SDS.

**Table 9a. Stability results of IL-2 mutants in 40 °C environment determined by SEC-HPLC**

| Sample name /buffer | 40 °C/0 days purity | 40 °C/7 days purity | 40 °C/14 days purity |
|---|---|---|---|
| Y089 /PBS | 99.80% | 99.41% | 99.32% |
| Y089/histidine | 99.64% | 99.23% | 99.23% |
| Y092 /PBS | 99.57% | 99.63% | 99.55% |
| Y092/histidine | 99.51% | 97.49% | 97.55% |
| Y094 /PBS | 99.62% | 99.47% | 99.33% |
| Y094/histidine | 99.58% | 99.22% | 98.96% |

**Table 9b. Stability results of IL-2 mutants in 40 °C environment determined by CE-SDS**

| Sample name /buffer | CE-SDS Purity | |
|---|---|---|
| | 40 °C/0 days | 40 °C/14 days |
| Y089 /PBS | 99.50% | 98.10% |
| Y089/histidine | 98.40% | 97.40% |
| Y092 /PBS | 99.40% | 98.40% |
| Y092/histidine | 97.70% | 90.70% |
| Y094 /PBS | 99.40% | 98.00% |
| Y094/histidine | 99.20% | 95.80% |

The results show that Y089, Y092 and Y094 all showed excellent stability after 14 days of storage in the PBS buffer and histidine buffer.

### Example 6: In Vivo Anti-tumor Efficacy of IL-2 Mutant Molecule

To demonstrate the *in vivo* efficacy of the IL-2 mutant molecule, Balb/c mice were inoculated with CT26 cells (mouse colon cancer cell line, ATCC) to determine the anti-tumor efficacy of the IL-2 mutant molecule (Y092) disclosed herein. SPF female Balb/c mice (18-20 g, purchased from Zhejiang Vital River Laboratory Animal Technology Co., Ltd.) with certificate No. 1811230011 were used in the experiment.

The CT26 cells were subcultured conventionally for subsequent *in vivo* study. The CT26 cells were collected by centrifugation and dispersed in PBS (1×) to form a cell suspension with a cell concentration of 2.5 × 10⁶ cells/mL. On day 0, 0.2 mL of the cell suspension was subcutaneously inoculated into the right abdominal region of the Balb/c mice to establish CT26 tumor-bearing mouse models. 7 days after the tumor cell inoculation, the tumor volume in each mouse was measured. The mice were divided into groups of 6. The dosages and routes of administration are shown in Table 10.

**Table 10. Groups, and dosages and routes of administration in the in vivo experiment**

| Groups | Dosage of administration | Number of administration | Route of administration |
|---|---|---|---|
| h-IgG* | 0.5mg/kg | Q7Dx2 | Intraperitoneal |
| Y092 | 0.004mg/kg | Q7Dx2 | Intraperitoneal |
| Y092 | 0.02mg/kg | Q7Dx2 | Intraperitoneal |
| Y092 | 0.1mg/kg | Q7Dx2 | Intraperitoneal |
| Y092 | 0.5mg/kg | Q7Dx2 | Intraperitoneal |

| | | | |
|---|---|---|---|
| *: h-IgG was an isotype control antibody, purchased from Equitech-Bio, lot No. 161206-0656. | | | |

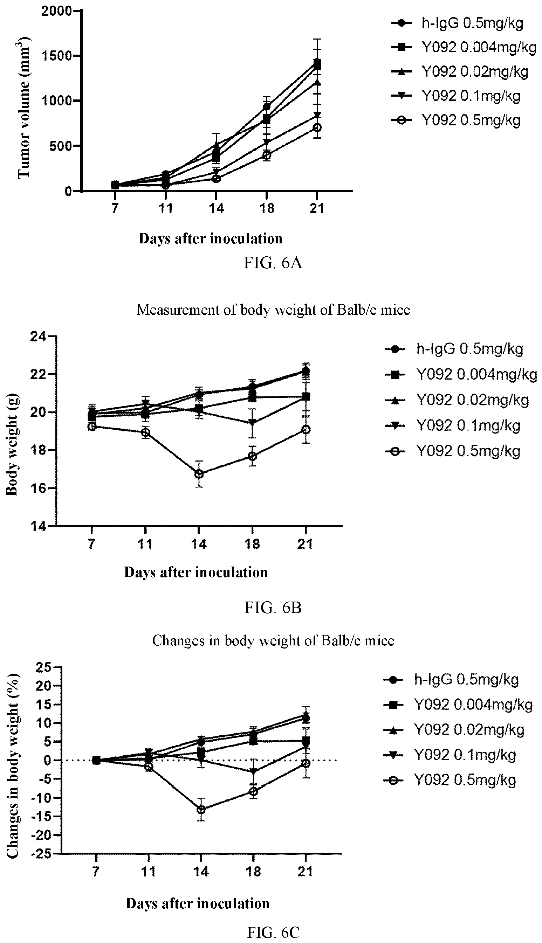

h-IgG and Y092 were used at concentrations of 10 mg/ml and 6.4 mg/ml, respectively, and administered every 7 days, for a total of 2 doses (Q7D × 2). Administration was performed on days 7 and 14 after the inoculation of CT26 cells. The tumor volume and body weight of the mice were monitored 2-3 times a week for 21 days, as shown in FIGs. 6A, 6B and 6C. On day 21 after inoculation, the relative tumor growth inhibition (TGI%) was calculated by the following formula: TGI% = 100% × (control group tumor volume - treatment group tumor volume)/(control group tumor volume - control group tumor volume before administration). Tumor volume measurement: The maximum length of major axis (L) and maximum length of minor axis (W) of tumors were measured with a vernier caliper, and tumor volume was calculated using the following formula: V = L × W²/2. The mice were weighted using an electronic balance.

The tumor growth inhibition results are shown in Table 11: on day 21 after inoculation, 0.004 mg/kg Y092, 0.02 mg/kg Y092, 0.1 mg/kg Y092 and 0.5 mg/kg Y092 had single-drug inhibition rates of 3.7%, 16.2%, 43.8% and 53.5%, respectively, compared to h-IgG. The results show that the engineered IL2 molecule (Y092) had an anti-tumor effect and achieved a dose-dependent response. Meanwhile, the results of the body weight measurement of mice (FIGs. 6B-6C) show that during the 21 days following the inoculation, a decrease of more than 10% occurred in the body weight in the high-dose group (Y092, 0.5 mg/kg), while no decrease of more than 5% occurred in the body weight in the other groups of mice. No death occurred in the administration groups of mice.

**Table 11. Tumor growth inhibition on day 21**

| Groups | Tumor volume (mm³) | Tumor growth inhibition (%) |
|---|---|---|
| h-IgG,0.5mg/kg | 1430.98 | N/A |
| Y092,0.004mg/kg | 1380.71 | 3.7 |
| Y092,0.02mg/kg | 1210.54 | 16.2 |
| Y092,0.1mg/kg | 834.57 | 43.8 |
| Y092,0.5mg/kg | 702.05 | 53.5 |

### Example 7: In Vivo Anti-tumor Efficacy of IL-2 Mutant Molecule

A mutation (T3A) was further performed in the molecule Y092 to reduce the glycosylation of the molecule. To demonstrate the *in vivo* efficacy of the IL-2 mutant molecule, C57 mice were inoculated with MC38 cells (mouse colon cancer cell line, ATCC) to determine the anti-tumor efficacy of the IL-2 mutant molecule (Y144) disclosed herein. SPF female C57 mice (15-18 g, purchased from Beijing Vital River Laboratory Animal Technology Co., Ltd.) with certificate No. 1100111911070497 were used in the experiment.

The MC38 cells were subcultured conventionally for subsequent *in vivo* study. The MC38 cells were collected by centrifugation and resuspended in PBS (1×) to form a cell suspension with a cell concentration of 5 × 10⁶ cells/mL. On day 0, 0.2 mL of the cell suspension was subcutaneously inoculated into the right abdominal region of the C57 mice to establish MC38 tumor-bearing mouse models. 7 days after the tumor cell inoculation, the tumor volume in each mouse was measured. The mice were divided into groups of 6. The dosages and routes of administration are shown in Table 12.

**Table 12. Groups, and dosages and routes of administration in the in vivo experiment**

| Groups | Dosage of administration | Number of administration | Route of administration |
|---|---|---|---|
| h-IgG* | 1mg/kg | Q7Dx3 | Intraperitoneal |
| Y144 | 0.5mg/kg | Q7Dx3 | Intraperitoneal |

| | | | |
|---|---|---|---|
| *: h-IgG was an isotype control antibody, purchased from Equitech-Bio, lot No. 161206-0656. | | | |

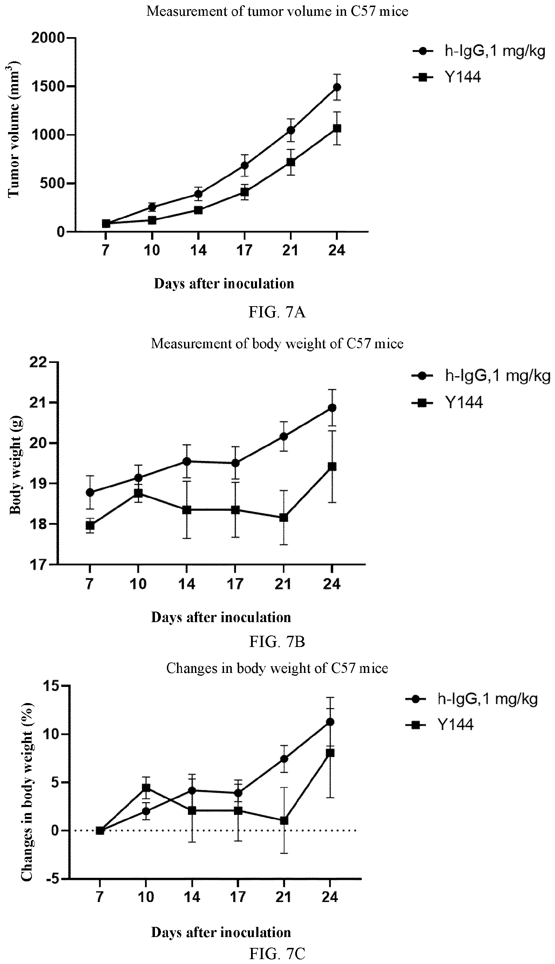

h-IgG and Y144 were used at concentrations of 10 mg/ml and 0.5 mg/ml, respectively, and administered every 7 days, for a total of 3 doses (Q7D × 3). Administration was performed on days 7, 14 and 21 after the inoculation of MC38 cells. The tumor volume and body weight of the mice were monitored twice a week for 24 days, as shown in FIGs. 7A, 7B and 7C. On day 24 after inoculation, the relative tumor growth inhibition (TGI%) was calculated by the following formula: TGI% = 100% × (control group tumor volume - treatment group tumor volume)/(control group tumor volume - control group tumor volume before administration). Tumor volume measurement: The maximum length of major axis (L) and maximum length of minor axis (W) of tumors were measured with a vernier caliper, and tumor volume was calculated using the following formula: V = L × W²/2. The mice were weighted using an electronic balance.

The tumor growth inhibition results are shown in Table 13: on day 24 after inoculation, Y144 had a single-drug inhibition rate of 30.05%, compared to the 1 mg/kg h-IgG group. Meanwhile, the results of the body weight measurement of mice (FIG. 7C) show that on day 24 after inoculation, there was no significant difference in the body weight of the mice.

### Sequence listing description

| SEQ ID NO | Name | Description | Sequences |
|---|---|---|---|
| 1 | Wild-type IL-2 | IL-2^{wt} with mutation C125S | APTSSSTKKTQLQLEHLLLDLQMILNGINNYKNPKLTRMLT FKFYMPKKATELKHLQCLEEELKPLEEVLNLAQSKNFHLR PRDLISNINVIVLELKGSETTFMCEYADETATIVEFLNRWIT FSQSIISTLT |
| 2 | Full-length human IL-2 | A native hIL-2 | MYRMQLLSCIALSLALVTNSAPTSSSTKKTQLQLEHLLLDL QMILNGINNYKNPKLTRMLTFKFYMPKKATELKHLQCLEE ELKPLEEVLNLAQSKNFHLRPRDLISNINVIVLELKGSETTF MCEYADETATIVEFLNRWITFCQSIISTLT |
| 3 | Mature human IL-2 | A native hIL-2 with signal peptides removed | |
| 4 | IL-2 mutant | IL-2^{3X} with mutations C125S, R38D, K43E and E61R | APTSSSTKKTQLQLEHLLLDLQMILNGINNYKNPKLTDML TFEFYMPKKATELKHLQCLERELKPLEEVLNLAQSKNFHL RPRDLISNINVIVLELKGSETTFMCEYADETATIVEFLNRWI TFSQSIISTLT |
| 5 | IL-2Rα receptor | An IL-2Rα receptor with an avi tag and an His6 | ELCDDDPPEIPHATFKAMAYKEGTMLNCECKRGFRRIKSG SLYMLCTGNSSHSSWDNQCQCTSSATRNTTKQVTPQPEEQ KERKTTEMQSPMQPVDQASLPGHCREPPPWENEATERIYH |
| | | tag at the C-terminus | FVVGQMVYYQCVQGYRALHRGPAESVCKMTHGKTRWT QPQLICTGEMETSQFPGEEKPQASPEGRPESETSCLVTTGLN DIFEAQKIEWHEHHHHHH |
| 6 | IL-2Rβ receptor | An IL-2Rβ receptor with an avi tag and an His6 tag at the C-terminus | AVNGTSQFTCFYNSRANISCVWSQDGALQDTSCQVHAWP DRRRWNQTCELLPVSQASWACNLILGAPDSQKLTTVDIVT LRVLCREGVRWRVMAIQDFKPFENLRLMAPISLQVVHVET HRCNISWEISQASHYFERHLEFEARTLSPGHTWEEAPLLTL KQKQEWICLETLTPDTQYEFQVRVKPLQGEFTTWSPWSQP LAFRTKPAALGKDTGLNDIFEAQKIEWHEHHHHHH |
| 7 | Mutant Fc region | A human IgG1 Fc with LALA mutation | DKTHTCPPCPAPEAAGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISKA KGQPREPQVYTLPPSREEMTKNQVSLTCLVKGFYPSDIAVE WESNGQPENNYKTTPPVLDSDGSFFLYSKLTVDKSRWQQ GNVFSCSVMHEALHNHYTQKSLSLSPGK |
| 8 | Y001 | IL-2-GSGS-FcLA LA | APTSSSTKKTQLQLEHLLLDLQMILNGINNYKNPKLTRMLT FKFYMPKKATELKHLQCLEEELKPLEEVLNLAQSKNFHLR PRDLISNINVIVLELKGSETTFMCEYADETATIVEFLNRWIT FSQSIISTLTGSGSDKTHTCPPCPAPEAAGGPSVFLFPPKPK DTLMISRTPEVTCVVVDVSHEDPEVKFNWYVDGVEVHNA KTKPREEQYNSTYRVVSVLTVLHQDWLNGKEYKCKVSNK ALPAPIEKTISKAKGQPREPQVYTLPPSREEMTKNQVSLTC LVKGFYPSDIAVEWESNGQPENNYKTTPPVLDSDGSFFLYS KLTVDKSRWQQGNVFSCSVMHEALHNHYTQKSLSLSPGK |
| 9 | Y002 | IL-^{2.3X}-GSGS-FcL ALA | APTSSSTKKTQLQLEHLLLDLQMILNGINNYKNPKLTDML TFEFYMPKKATELKHLQCLERELKPLEEVLNLAQSKNFHL RPRDLISNINVIVLELKGSETTFMCEYADETATIVEFLNRWI TFSQSIISTLTGSGSDKTHTCPPCPAPEAAGGPSVFLFPPKPK DTLMISRTPEVTCVVVDVSHEDPEVKFNWYVDGVEVHNA KTKPREEQYNSTYRVVSVLTVLHQDWLNGKEYKCKVSNK ALPAPIEKTISKAKGQPREPQVYTLPPSREEMTKNQVSLTC LVKGFYPSDIAVEWESNGQPENNYKTTPPVLDSDGSFFLYS KLTVDKSRWQQGNVFSCSVMHEALHNHYTQKSLSLSPGK |
| 10 | Y017 | IL-2^{hyb15BCL}-GSGS -FcLALA | APTSSSTKKTQLQLEHLLLDLQMILNGINNYKNPKLTRMLT FKFYMPKKATELKHLQCLEEELKPLEEVLNLSGDASIHDLI SNINVIVLELKGSETTFMCEYADETATIVEFLNRWITFSQSII STLTGSGSDKTHTCPPCPAPEAAGGPSVFLFPPKPKDTLMIS RTPEVTCVVVDVSHEDPEVKFNWYVDGVEVHNAKTKPRE |
| | | | EQYNSTYRVVSVLTVLHQDWLNGKEYKCKVSNKALPAPI EKTISKAKGQPREPQVYTLPPSREEMTKNQVSLTCLVKGF YPSDIAVEWESNGQPENNYKTTPPVLDSDGSFFLYSKLTVD KSRWQQGNVFSCSVMHEALHNHYTQKSLSLSPGK |
| 11 | Y057 | IL-2^{truncate1}-GSGS-FcLALA | APTSSSTKKTQLQLEHLLLDLQMILNGINNYKNPKLTRMLT FKFYMPKKATELKHLQCLEEELKPLEEVLNLAQSKNFHDL ISNINVIVLELKGSETTFMCEYADETATIVEFLNRWITFSQSI ISTLTGSGSDKTHTCPPCPAPEAAGGPSVFLFPPKPKDTLMI SRTPEVTCVVVDVSHEDPEVKFNWYVDGVEVHNAKTKPR EEQYNSTYRVVSVLTVLHQDWLNGKEYKCKVSNKALPAP IEKTISKAKGQPREPQVYTLPPSREEMTKNQVSLTCLVKGF YPSDIAVEWESNGQPENNYKTTPPVLDSDGSFFLYSKLTVD KSRWQQGNVFSCSVMHEALHNHYTQKSLSLSPGK |
| 12 | Y058 | IL-2^{truncate2}-GSGS-FcLALA | APTSSSTKKTQLQLEHLLLDLQMILNGINNYKNPKLTRMLT FKFYMPKKATELKHLQCLEEELKPLEEVLNLAGSKNFHDL ISNINVIVLELKGSETTFMCEYADETATIVEFLNRWITFSQSI ISTLTGSGSDKTHTCPPCPAPEAAGGPSVFLFPPKPKDTLMI SRTPEVTCVVVDVSHEDPEVKFNWYVDGVEVHNAKTKPR EEQYNSTYRVVSVLTVLHQDWLNGKEYKCKVSNKALPAP IEKTISKAKGQPREPQVYTLPPSREEMTKNQVSLTCLVKGF YPSDIAVEWESNGQPENNYKTTPPVLDSDGSFFLYSKLTVD KSRWQQGNVFSCSVMHEALHNHYTQKSLSLSPGK |
| 13 | Y059 | IL-2 ^{truncate3}-GSGS-FcL ALA | APTSSSTKKTQLQLEHLLLDLQMILNGINNYKNPKLTRMLT FKFYMPKKATELKHLQCLEEELKPLEEVLNLAQSANFHDL ISNINVIVLELKGSETTFMCEYADETATIVEFLNRWITFSQSI ISTLTGSGSDKTHTCPPCPAPEAAGGPSVFLFPPKPKDTLMI SRTPEVTCVVVDVSHEDPEVKFNWYVDGVEVHNAKTKPR EEQYNSTYRVVSVLTVLHQDWLNGKEYKCKVSNKALPAP IEKTISKAKGQPREPQVYTLPPSREEMTKNQVSLTCLVKGF YPSDIAVEWESNGQPENNYKTTPPVLDSDGSFFLYSKLTVD KSRWQQGNVFSCSVMHEALHNHYTQKSLSLSPGK |
| 14 | Y060 | IL-2 ^{truncate4}-GSGS-FcL ALA | APTSSSTKKTQLQLEHLLLDLQMILNGINNYKNPKLTRMLT FKFYMPKKATELKHLQCLEEELKPLEEVLNLAQSANIHDLI SNINVIVLELKGSETTFMCEYADETATIVEFLNRWITFSQSII STLTGSGSDKTHTCPPCPAPEAAGGPSVFLFPPKPKDTLMIS RTPEVTCVVVDVSHEDPEVKFNWYVDGVEVHNAKTKPRE EQYNSTYRVVSVLTVLHQDWLNGKEYKCKVSNKALPAPI EKTISKAKGQPREPQVYTLPPSREEMTKNQVSLTCLVKGF YPSDIAVEWESNGQPENNYKTTPPVLDSDGSFFLYSKLTVD |
| | | | KSRWQQGNVFSCSVMHEALHNHYTQKSLSLSPGK |
| 15 | Y089 | IL-2^{.Y30E1.truncate1}-2 *(G4S)-FcLALA | APTSSSTKKTQLQLEHLLLDLQMILNGINNYKNPELEEMLT AKFYMPKKATELKHLQCLEEELKPLEEVLNLAQSKNFHDL ISNINVIVLELKGSETTFMCEYADETATIVEFLNRWITFSQSI ISTLTGGGGSGGGGSDKTHTCPPCPAPEAAGGPSVFLFPPK PKDTLMISRTPEVTCVVVDVSHEDPEVKFNWYVDGVEVH NAKTKPREEQYNSTYRVVSVLTVLHQDWLNGKEYKCKVS NKALPAPIEKTISKAKGQPREPQVYTLPPSREEMTKNQVSL TCLVKGFYPSDIAVEWESNGQPENNYKTTPPVLDSDGSFFL YSKLTVDKSRWQQGNVFSCSVMHEALHNHYTQKSLSLSP GK |
| 16 | Y092 | IL-2 ^{Y30E1.15BCL}-2^{∗}(G4S )-FcLALA | APTSSSTKKTQLQLEHLLLDLQMILNGINNYKNPELEEMLT AKFYMPKKATELKHLQCLEEELKPLEEVLNLAGDASIHDLI SNINVIVLELKGSETTFMCEYADETATIVEFLNRWITFSQSII STLTGGGGSGGGGSDKTHTCPPCPAPEAAGGPSVFLFPPKP KDTLMISRTPEVTCVVVDVSHEDPEVKFNWYVDGVEVHN AKTKPREEQYNSTYRVVSVLTVLHQDWLNGKEYKCKVSN KALPAPIEKTISKAKGQPREPQVYTLPPSREEMTKNQVSLT CLVKGFYPSDIAVEWESNGQPENNYKTTPPVLDSDGSFFL YSKLTVDKSRWQQGNVFSCSVMHEALHNHYTQKSLSLSP GK |
| 17 | Y093 | IL-2^{.Y30E1.truncate1.K7 6A}-2^{∗}(G4S)-FcLA LA | APTSSSTKKTQLQLEHLLLDLQMILNGINNYKNPELEEMLT AKFYMPKKATELKHLQCLEEELKPLEEVLNLAQSANFHDL ISNINVIVLELKGSETTFMCEYADETATIVEFLNRWITFSQSI ISTLTGGGGSGGGGSDKTHTCPPCPAPEAAGGPSVFLFPPK PKDTLMISRTPEVTCVVVDVSHEDPEVKFNWYVDGVEVH NAKTKPREEQYNSTYRVVSVLTVLHQDWLNGKEYKCKVS NKALPAPIEKTISKAKGQPREPQVYTLPPSREEMTKNQVSL TCLVKGFYPSDIAVEWESNGQPENNYKTTPPVLDSDGSFFL YSKLTVDKSRWQQGNVFSCSVMHEALHNHYTQKSLSLSP GK |
| 18 | Y094 | IL-2^{.Y30E1.truncate1.K7 6D}-2^{∗}(G4S)-FcLA LA | APTSSSTKKTQLQLEHLLLDLQMILNGINNYKNPELEEMLT AKFYMPKKATELKHLQCLEEELKPLEEVLNLAQSDNFHDL ISNINVIVLELKGSETTFMCEYADETATIVEFLNRWITFSQSI ISTLTGGGGSGGGGSDKTHTCPPCPAPEAAGGPSVFLFPPK PKDTLMISRTPEVTCVVVDVSHEDPEVKFNWYVDGVEVH NAKTKPREEQYNSTYRVVSVLTVLHQDWLNGKEYKCKVS NKALPAPIEKTISKAKGQPREPQVYTLPPSREEMTKNQVSL TCLVKGFYPSDIAVEWESNGQPENNYKTTPPVLDSDGSFFL |
| | | | YSKLTVDKSRWQQGNVFSCSVMHEALHNHYTQKSLSLSP GK |
| 19 | Y144 | IL-2 ^{Y30E1.15BCL.T3A}-2^{∗}( G4S)-FcLALA | APASSSTKKTQLQLEHLLLDLQMILNGINNYKNPELEEMLT AKFYMPKKATELKHLQCLEEELKPLEEVLNLAGDASIHDLI SNINVIVLELKGSETTFMCEYADETATIVEFLNRWITFSQSII STLTGGGGSGGGGSDKTHTCPPCPAPEAAGGPSVFLFPPKP KDTLMISRTPEVTCVVVDVSHEDPEVKFNWYVDGVEVHN AKTKPREEQYNSTYRVVSVLTVLHQDWLNGKEYKCKVSN KALPAPIEKTISKAKGQPREPQVYTLPPSREEMTKNQVSLT CLVKGFYPSDIAVEWESNGQPENNYKTTPPVLDSDGSFFL YSKLTVDKSRWQQGNVFSCSVMHEALHNHYTQKSLSLSP GK |
| 20 | Y040 | IL-^{2.3X}-2^{∗}(G4S)-Fc LALA | APTSSSTKKTQLQLEHLLLDLQMILNGINNYKNPKLTDML TFEFYMPKKATELKHLQCLERELKPLEEVLNLAQSKNFHL RPRDLISNINVIVLELKGSETTFMCEYADETATIVEFLNRWI TFSQSIISTLTGGGGSGGGGSDKTHTCPPCPAPEAAGGPSVF LFPPKPKDTLMISRTPEVTCVVVDVSHEDPEVKFNWYVDG VEVHNAKTKPREEQYNSTYRVVSVLTVLHQDWLNGKEY KCKVSNKALPAPIEKTISKAKGQPREPQVYTLPPSREEMTK NQVSLTCLVKGFYPSDIAVEWESNGQPENNYKTTPPVLDS DGSFFLYSKLTVDKSRWQQGNVFSCSVMHEALHNHYTQK SLSLSPGK |
| 21 | Y045 | IL-2^{WT}-2*(G4S)-F cLALA | APTSSSTKKTQLQLEHLLLDLQMILNGINNYKNPKLTRMLT FKFYMPKKATELKHLQCLEEELKPLEEVLNLAQSKNFHLR PRDLISNINVIVLELKGSETTFMCEYADETATIVEFLNRWIT FSQSIISTLTGGGGSGGGGSDKTHTCPPCPAPEAAGGPSVFL FPPKPKDTLMISRTPEVTCVVVDVSHEDPEVKFNWYVDGV EVHNAKTKPREEQYNSTYRVVSVLTVLHQDWLNGKEYKC KVSNKALPAPIEKTISKAKGQPREPQVYTLPPSREEMTKNQ VSLTCLVKGFYPSDIAVEWESNGQPENNYKTTPPVLDSDG SFFLYSKLTVDKSRWQQGNVFSCSVMHEALHNHYTQKSL SLSPGK |
| 22 | Combinator ial mutant | IL-2^{.Y30E1.truncate1} | APTSSSTKKTQLQLEHLLLDLQMILNGINNYKNPELEEMLT AKFYMPKKATELKHLQCLEEELKPLEEVLNLAQSKNFHDL ISNINVIVLELKGSETTFMCEYADETATIVEFLNRWITFSQSI ISTLT |
| 23 | Combinator ial mutant | IL-2^{.Y30E1.15BCL} | APTSSSTKKTQLQLEHLLLDLQMILNGINNYKNPELEEMLT AKFYMPKKATELKHLQCLEEELKPLEEVLNLAGDASIHDLI |
| | | | SNINVIVLELKGSETTFMCEYADETATIVEFLNRWITFSQSII STLT |
| 24 | Combinator ial mutant | IL-2^{.Y30E1.truncate1.K7 6A} | APTSSSTKKTQLQLEHLLLDLQMILNGINNYKNPELEEMLT AKFYMPKKATELKHLQCLEEELKPLEEVLNLAQSANFHDL ISNINVIVLELKGSETTFMCEYADETATIVEFLNRWITFSQSI ISTLT |
| 25 | Combinator ial mutant | IL-2^{.Y30E1.truncate1.K7 6D} | APTSSSTKKTQLQLEHLLLDLQMILNGINNYKNPELEEMLT AKFYMPKKATELKHLQCLEEELKPLEEVLNLAQSDNFHDL ISNINVIVLELKGSETTFMCEYADETATIVEFLNRWITFSQSI ISTLT |
| 26 | Combinator ial mutant | IL-2^{. Y30E1.15BCL.T3A} | APASSSTKKTQLQLEHLLLDLQMILNGINNYKNPELEEMLT AKFYMPKKATELKHLQCLEEELKPLEEVLNLAGDASIHDLI SNINVIVLELKGSETTFMCEYADETATIVEFLNRWITFSQSII STLT |

## Claims

1. An IL-2 mutant protein, compared to a wild-type IL-2 (preferably a human IL-2, and more preferably an IL-2 comprising a sequence of SEQ ID NO: 1), comprising mutations:
(i) a mutation that eliminates or reduces the binding affinity for an IL-2α receptor, on a binding interface of IL-2 to CD25, particularly at at least one position selected from positions 35, 37, 38, 41, 42, 43, 45, 61, 68 and 72, wherein preferably, the mutation is selected from any one of K35D/E, T37D/E, R38Y/W/E/D, T41E, F42N/Q/V/L/I/A, K43D/E/F/Y, Y45R/K, E61R/K, E68R/K and L72Y/F or any combination thereof, more preferably from any one of K35D/E, T37D/E, R38W/E/D, T41E, F42Q/A, K43E/Y, Y45K, E61K, E68R and L72F or any combination thereof;
and
(ii) a shortened B'C' loop region (i.e., a sequence linking amino acid residues aa72 and aa84), wherein preferably, the shortened loop region has less than 10, 9, 8, 7, 6 or 5 amino acids in length, and more preferably has 7 amino acids in length; preferably, the shortened B'C' loop region leads to an improved protein expression yield and/or purity and/or binding affinity for an IL-2RP receptor,
wherein, the amino acid positions are numbered according to SEQ ID NO: 1.

2. The mutant protein according to claim 1, wherein the mutation (i) is a combination of mutations selected from the following combinations (1)-(9), particularly from the combinations (1)-(6):
| Combinations | Mutations |
|---|---|
| 1 | K35D/**E**+T37E/**D**+R38Y/**W**+F42N/**Q**+Y45R/**K**+E61R/**K**+E68K/**R**, preferably K35D/**E**+T37E/**D**+R38**W**+F42**Q**+Y45R/**K**+E61R/**K**+E68K/**R**, and more preferably K35D/**E**+T37E/**D**+R38**W**+F42**Q**+Y45**K**+E61**K**+E68**R** |
| 2 | K35D/**E**+T37D/**E**+R38D/**E**+F42V/L/I/**A**, preferably K35D/**E**+T37D/**E**+R38D/**E**+F42V/**A**, and more preferably K35D/**E**+T37D/**E**+R38D/**E**+F42**A** |
| 3 | K35E/**D**+R38D/**E**+T41D/**E**+K43D/**E**, preferably K35E/**D**+R38D/**E**+T41**E**+K43**E** |
| 4 | K35E/**D**+T37D/**E**+R38E/**D**+K43F/**Y**+Y45R/**K**+L72Y/**F**, more preferably K35E/**D**+T37D/**E**+R38E/**D**+K43**Y**+Y45**K**+L72**F** |
| 5 | K35D/**E**+R38D/**E**+T41D/**E**+K43F/**Y**+Y45R/**K**+L72Y/**F**, more preferably K35D/**E**+R38D/**E**+T41D/**E**+K43**Y**+Y45**K**+L72**F** |
| 6 | K35E/**D**+T37D/**E**+R38E/**D**+T41D/**E**+K43D/**E**+L72Y/**F**, more preferably K35E/**D**+T37D/**E**+R38E/**D**+T41D/**E**+K43D/**E**+L72**F** |
| 7 | K35E/**D**+T37D/**E**+R38E/**D**+K43D/**E**+L72Y/**F**, more preferably K35E/**D**+T37D/**E**+R38E/**D**+K43D/**E**+L72**F** |
| 8 | K35D/**E**+T37E/**D**+R38E/**D**+K43D/**E**+L72Y/**F**, more preferably K35D/**E**+T37E/**D**+R38E/**D**+K43D/**E**+L72**F** |
| 9 | K35D/**E**+R38E/**D**+T41D/**E**+K43D/**E**+E61R/**K**+L72Y/**F**, more preferably K35D/**E**+R38E/**D**+T41D/**E**+K43D/**E**+E61**K**+L72**F** |
preferably, the mutation (i) is a combination of mutations selected from the following combinations (1)-(9), particularly from the combinations (1)-(6):
| Combinations | Mutations |
|---|---|
| 1 | K35**E**+T37**D**+R38**W**+F42**Q**+Y45**K**+E61**K**+E68**R** |
| 2 | K35**E**+T37**E**+R38**E**+F42**A** |
| 3 | K35**D**+R38**E**+T41**E**+K43**E** |
| 4 | K35**D**+T37**E**+R38**D**+K43**Y**+Y45**K**+L72**F** |
| 5 | K35**E**+R38**E**+T41**E**+K43**Y**+Y45**K**+L72**F** |
| 6 | K35**D**+T37**E**+R38**D**+T41**E**+K43**E**+L72**F** |
| 7 | K35**D**+T37**E**+R38**D**+K43**E**+L72**F** |
| 8 | K35**E**+T37**D**+R38**D**+K43**E**+L72**F** |
| 9 | K35**E**+R38**D**+T41**E**+K43**E**+E61**K**+L72**F** |

3. The mutant protein according to claim 1, comprising a combination of mutations K35**E**+T37**E**+R38**E**+F42**A**.

4. The mutant protein according to claim 1, wherein the mutation (ii) comprises:
(a) a substitution of aa74 to aa83 in a B'C' loop region, for example, with a short B'C' loop sequence from four-helical short-chain cytokine IL family members, such as a B'C' loop sequence from IL-15, preferably, with a sequence GDASIH; or
(b) a truncation of aa74 to aa83 in a B'C' loop region, for example, by 1, 2, 3 or 4 amino acids at the C-terminus; preferably a truncation to form a sequence (Q/G)S(K/A/D)N(F/I)H, and more preferably a truncation to form a sequence selected from the following:
| |
|---|
| QSKNFH |
| QSANFH |
| QSDNFH |
| GSKNFH |
| QSANFH |
| QSANIH |

5. The mutant protein according to claim 1, relative to the wild-type IL-2, comprising:
(i) a combination of mutations K35**E**+T37**E**+R38**E**+F42**A**; and
(ii) a B'C' loop region sequence selected from:
- AQSKNFH;
- AQSANFH;
- AQSDNFH;
- SGDASIH; and
- AGDASIH,
and optionally, (iii) a mutation T3A.

6. The mutant protein according to any one of claims 1-5, relative to the wild-type IL-2, further comprising no more than 0-5 amino acid mutations, such as conservative amino acid residue mutations.

7. The mutant protein according to any one of claims 1-6, comprising:
- an amino acid sequence having at least 90%, 92%, 93%, 94%, 95%, 96%, 97% or 98% identity to an amino acid sequence selected from SEQ ID NOs: 22-26, preferably from SEQ ID NOs: 23 and 26;
- an amino acid sequence selected from SEQ ID NOs: 22-26, preferably SEQ ID NO: 23 or 26.

8. The mutant protein according to any one of claims 1-7, compared to the wild-type IL-2, comprising at least one, two or all three of the following properties:
- reduced or eliminated binding to IL-2Rα,
- enhanced binding to IL-2Rβ; and
- improved expression yield and/or ease of purification to higher purity (e.g. higher purity as measured by SEC-HPLC after one-step affinity chromatography) when expressed in mammalian cells (e.g. 293 cells or CHO cells), e.g. as an Fc fusion protein.

9. The mutant protein according to any one of claims 1-8, compared to the wild-type IL-2, having one or more of the following properties:
- having reduced binding affinity for a high-affinity IL-2R receptor (IL-2Rαβγ);
- having increased binding affinity for an intermediate-affinity IL-2R receptor (IL-2Rβγ);
- reducing activation of CD25⁺ cells (e.g., CD8⁺ T cells and Treg cells);
- reducing stimulation of IL-2-mediated signaling in CD25⁺ cells (e.g., CD8⁺ T cells);
- eliminating or reducing preference of an IL-2 for preferentially activating CD25⁺ cells (e.g., Treg cells);
- reducing Treg-associated immune response downregulation induced by IL-2;
- maintaining or enhancing activation of CD25⁻ cells (e.g., CD25⁻ CD8⁺ T cells); and
- leading to increased proliferation and activation of CD25⁻ effector T cells and NK cells;
wherein optionally, the mutant protein further has one or more of the following properties:
- an *in vivo* anti-tumor effect, e.g., on colon cancer;
- no significant toxicity after being administered *in vivo,* as reflected, e.g., by changes in the body weight of a subject after being administered (e.g., a reduction of no more than 15% or no more than 10% or no more than 5% in the body weight, compared to that before administration, e.g., 20 days after administration); and
- having storage stability, wherein e.g., after being stored at 40 °C in PBS buffer at pH 7.4 for 14 days, the protein has a decrease of no more than 2% or 1% in purity as measured by SEC-HPLC, or has a decrease of no more than 3% or 2% in purity as measured by CE-SDS.

10. A fusion protein of IL-2 mutant protein, comprising the IL2 mutant protein according to any one of claims 1-9, wherein, preferably, the IL-2 mutant protein is fused to an Fc antibody fragment, and more preferably, the IL-2 mutant protein is fused to the Fc through a linker, the linker being preferably GSGS and more preferably 2 × (G4S), wherein the Fc is human IgG1 Fc, and preferably comprises mutations that reduce or eliminate binding of the Fc to FcyR, e.g., L234A+L235A;
preferably, the fusion protein comprises a sequence having at least 85%, at least 95%, at least 96% or 100% identity to an amino acid sequence selected from SEQ ID NOs: 15-19.

11. An immunoconjugate, comprising the IL2 mutant protein according to any one of claims 1-9 and an antigen-binding molecule, wherein preferably, the antigen-binding molecule is an immunoglobulin molecule, particularly an IgG molecule, or an antibody or an antibody fragment, and more particularly an Fab molecule and an scFv molecule.

12. The immunoconjugate according to claim 11, wherein the antigen-binding molecule specifically binds to an antigen present on a tumor cell or in tumor environment, such as an antigen selected from: fibroblast activation protein (FAP), A1 domain of tenascin-C (TNC A1), A2 domain of tenascin-C (TNC A2), extra domain B (EDB) of fibronectin, carcinoembryonic antigen (CEA), and melanoma-associated chondroitin sulfate proteoglycan (MCSP).

13. An isolated polynucleotide, encoding the IL-2 mutant protein according to any one of claims 1-9, the fusion according to claim 10, or the immunoconjugate according to any one of claims 11-12.

14. An expression vector, comprising the polynucleotide according to claim 13.

15. A host cell, comprising the polynucleotide according to claim 13 or the vector according to claim 14, wherein preferably, the host cell is a mammalian cell, particularly an HEK293 cell or a CHO cell, or a yeast.

16. A method for producing an IL-2 mutant protein, or a fusion or an immunoconjugate thereof, comprising culturing the host cell according to claim 15 under a condition suitable for expressing the IL-2 mutant protein, or the fusion or the conjugate thereof.

17. A pharmaceutical composition, comprising the IL-2 mutant protein according to any one of claims 1-9, the fusion according to claim 10, or the immunoconjugate according to any one of claims 11-12, and a pharmaceutically acceptable carrier, wherein preferably, the composition comprises a phosphate buffer or a histidine buffer; preferably the composition has a pH of 6.0-7.6.

18. A method for treating a disease in a subject, comprising administering to the subject the IL-2 mutant protein according to any one of claims 1-9, the fusion according to claim 10, the immunoconjugate according to any one of claims 11-12, or the pharmaceutical composition according to claim 17, wherein preferably, the disease is cancer.

19. A method for stimulating the immune system of a subject, comprising administering to the subject an effective amount of a pharmaceutical composition comprising the IL-2 mutant protein according to any one of claims 1-9, the fusion according to claim 10, or the immunoconjugate according to any one of claims 11-12.

20. A method for obtaining an IL-2 mutant protein, comprising the following the steps:
- introducing one or more mutations by performing mutation at the binding interface of IL-2 to IL-2Rα, and shortening the sequence of the B'C' loop region of the IL-2 by mutation therein,
preferably introducing the combination of mutations described in claim 2 and/or the mutation of the B'C' loop sequence described in claim 4;
- expressing the IL-2 mutant protein in a mammalian cell (e.g., an HEK293 or CHO cell), for example, in the form of an Fc fusion (e.g., an FcLALA fusion); and
- identifying a mutant protein having one or more of the following improved properties: (i) improved expression yield and/or protein purity after purification (e.g., purity as measured by SEC-HPLC after one-step affinity chromatography); (ii) reduced binding to IL2Rα; and (iii) enhanced binding to IL2Rβ.

21. A method for engineering a B'C' loop region of an IL-2 protein, comprising:
(a) substituting aa74 to aa83 in the B'C' loop region, for example, with a short B'C' loop sequence from four-helical short-chain cytokine IL family members, such as a B'C' loop sequence from IL-15, preferably, with a sequence including GDASIH; or
(b) substituting aa73 to aa83 in the B'C' loop region, for example, with a short B'C' loop sequence from four-helical short-chain cytokine IL family members, such as a B'C' loop sequence from IL-15, preferably, with a sequence including AGDASIH; or
(c) truncating aa74 to aa83 in the B'C' loop region, for example, by 1, 2, 3 or 4 amino acids at the C-terminus; preferably to form a sequence (Q/G)S(K/A/D)N(F/I)H, and more preferably to form a sequence selected from the following:
| |
|---|
| QSKNFH |
| QSANFH |
| QSDNFH |
| GSKNFH |
| QSANFH |
| QSANIH |

22. An IL-2 protein, engineered by using the method according to claim 21.
